(19) Europäisches Patentamt
European Patent Office
Office européen des brevets

(11) EP 1 792 898 A1

(12) **EUROPEAN PATENT APPLICATION**
published in accordance with Art. 158(3) EPC

(43) Date of publication:
**06.06.2007 Bulletin 2007/23**

(21) Application number: **05781555.7**

(22) Date of filing: **01.09.2005**

(51) Int Cl.:
*C07D 239/94* (2006.01) *A61K 31/517* (2006.01)
*A61P 13/08* (2006.01) *A61P 15/00* (2006.01)
*A61P 35/00* (2006.01) *A61P 35/02* (2006.01)
*A61P 43/00* (2006.01)

(86) International application number:
**PCT/JP2005/016007**

(87) International publication number:
**WO 2006/025490 (09.03.2006 Gazette 2006/10)**

(84) Designated Contracting States:
**AT BE BG CH CY CZ DE DK EE ES FI FR GB GR HU IE IS IT LI LT LU LV MC NL PL PT RO SE SI SK TR**

(30) Priority: **01.09.2004 JP 2004254716**

(71) Applicant: **Mitsubishi Pharma Corporation**
**Osaka-shi,**
**Osaka 541-0046 (JP)**

(72) Inventors:
- **SUZUKI, Tsuyoshi,**
**c/o MITSUBISHI PHARMA CORP.**
**Tokyo 1038405 (JP)**
- **FUJII, Akihiro,**
**c/o MITSUBISHI PHARMA CORP.**
**Tokyo 1038405 (JP)**
- **NAKAMURA, Hideo,**
**c/o MITSUBISHI PHARMA CORP.**
**Tokyo 1038405 (JP)**
- **YOSHIKAWA, Tsutomu,**
**c/o MITSUBISHI PHARMA CORP.**
**Tokyo 1038405 (JP)**

(74) Representative: **von Kreisler, Alek et al**
**Deichmannhaus am Dom,**
**Postfach 10 22 41**
**50462 Köln (DE)**

(54) **MOLECULAR CHAPERONE FUNCTION REGULATOR**

(57) According to the present invention, a molecular chaperone function regulator containing a quinazoline derivative represented by the following formula (I)

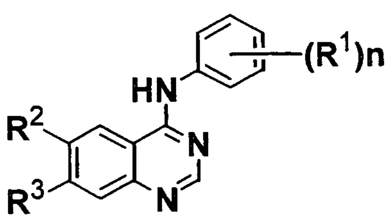

( I )

wherein each symbol is as defined in Claims, or a pharmaceutically acceptable salt thereof, a hydrate or solvate thereof, an optically active form or racemate thereof or a diastereomer mixture thereof as an active ingredient can be provided.

EP 1 792 898 A1

## Description

### Technical Field

The present invention relates to the prophylaxis and/or treatment of a disease involving a heat shock protein, which uses, as an active ingredient, a compound regulating the molecular chaperone function.

### Background Art

Heat shock proteins (hereinafter sometimes indicated as HSP) are constitutively expressed in cells, and are responsible for the molecular chaperone function such as folding of and transport to the organelle of newly translated and synthesized proteins. Moreover, when cells are exposed to a stress, HSP is induced to express and prevents denaturation of proteins, or when the denaturation is remarkable, promote degradation of denatured proteins.

While examples of HSP are not limited, HSP70, HSP90 and the like can be mentioned. For example, the chaperone function of HSP90 does not operate alone but operates in conjunction with cochaperones such as HSP40, HSP70 and the like. The function is regulated by a complicated process where plural cochaperones mediate and exchange in stages during progress.

HSP90 forms a complex in a cell with various kinds of proteins and, in many cases, sustained interactions with HSP90 are essential for the stable presence and maintenance of normal functions of target proteins (hereinafter sometimes referred to as client proteins). The client proteins of HSP90 characteristically include many signaling molecules that play important roles in cell growth and cell differentiation, such as protein kinases, steroid hormone receptors and the like. Theoretically, therefore, a pharmaceutical agent that inhibits the molecular chaperone function of HSP90 also simultaneously deactivates many client proteins in the cell, and is expected to be effective for cell proliferative diseases, for example, malignant tumor.

Among the malignant tumors, particularly in breast cancer, prostate cancer and endometrial cancer that grow in a hormone-dependent manner, since both the steroid hormone receptors (receptor groups of estrogen, progesterone, androgen and the like) and cell growth-related kinases (ErbB2, Raf, Akt, Cdc2, Cdk4 etc.), included in the above-mentioned client proteins, have important functions for the survival and maintenance of the cancer cells thereof, a molecular chaperone function regulator is considered to have a high possibility of most effectively exhibiting the action. It is also considered that not only hormone-sensitive cancers but also hormone-dependent diseases such as prostatic hyperplasia, endometriosis and the like will be suitable targets of a molecular chaperone function regulator.

A determining gene responsible for chronic myelocytic leukemia, which is a representative hematopoietic tumor, is known to be a tyrosine kinase, bcr-abl, and this kinase is also a client protein of HSP90. Moreover, there is a report concluding that an HSP90 inhibitor causes apoptosis in bcr-abl positive leukemia cells and also promotes differentiation (see non-patent reference 1). Therefore, hematopoietic tumor is considered to be one of suitable target diseases for a molecular chaperone regulator.

In general, induction of molecular chaperone is said to affect survival and maintenance of tumor cells. For example, a report has documented that HSP90 shows a constitutive increase in the expression amount in various tumor cells than normal cells (see non-patent reference 2). Since HSP90 expressed in tumor cell characteristically forms a functionally more highly active chaperone complex than in normal cells, HSP90 has also been reported to be a potential object in the development of an anticancer agent (see non-patent reference 3). With regard to HSP, a clinical report reveals that the disease-free survival time is significantly short and the prognosis is very poor in patients with breast cancer who highly expressed HSP70 (see non-patent reference 4). Currently, therefore, there is a demand for a highly useful therapeutic drug or preventive drug for the treatment of malignant tumor.

A cancer thermotherapy utilizes the property of cancer cells in that they are weak against heat as compared to normal cells, and warm the whole body or tumor lesion to not less than 41°C. In general, thermotherapy is not used alone but often applied in combination with radiation or anticancer agents, in an attempt to enhance the respective treatment effects. The kind of cancer for which clinical study of thermotherapy has been actively undertaken includes malignant melanoma and soft tissue sarcoma. Soft tissue sarcoma is developed from comparatively young age and its frequent site is extremities. For induction therapy for conserving surgery of diseased limb and suppression of metastasis, clinical tests using thermotherapy and anticancer agents in combination have been vigorously reviewed (see non-patent reference 5). Accordingly, soft tissue sarcoma is considered to be one of the most suitable kinds of cancer to which to apply a hyperthermic enhancer.

When thermotherapy is repeated, tumor cells may acquire thermo tolerance to attenuate the effect. Induction of HSP70, HSP110 and the like in tumor cells reportedly relates to the acquisition of thermo tolerance and stress resistance (see non-patent reference 6). Accordingly, a pharmaceutical agent that regulates the chaperone function is expected to remove thermo tolerance of tumor cells and enhance the hyperthermic effect, thereby efficiently inhibiting the growth of tumor cells. However, almost no report exists on the study relating to the effect of hyperthermic enhancement

by the chaperone regulator, and further research and development are necessary for the establishment of an effective treatment method utilizing hyperthermic enhancement.

Conventionally, benzoquinone ansamycin compounds such as herbimycin A, geldanamycin and the like are known to bind to HSP90 (see non-patent reference 7). 17-Allylamino-17-demethoxygeldanamycin (hereinafter to be indicated as 17-AAG), which is a geldanamycin derivative, is currently under clinical trials, and its effectiveness and safety have not been established.

Accordingly, a compound sold as a pharmaceutical product based on the action on HSP90 does not exist yet. Particularly, there are few compounds in the stage of clinical development of oral preparation at present.

Geldanamycin and 17-AAG are known to suppress expression of client proteins of HSP90 in tumor cells and also simultaneously increase expression of HSP70. This is considered to be the results of the conformational change from an HSP90-client complex to an HSP70-client complex prior to the proteosome degradation of client proteins (see non-patent reference 1).

On the contrary, no report has directly demonstrated regulation of molecular chaperone function by a compound having a quinazoline skeleton structure, and no finding suggestive thereof is known.

non-patent reference 1: Cancer Res. 61, 1799-804, 2001
non-patent reference 2: Int. J. Cancer 51, 613-9, 1992
non-patent reference 3: Nature 425, 407-10, 2003
non-patent reference 4: J. Natl. Cancer Inst. 85, 570-4, 1993
non-patent reference 5: Clin. Cancer Res. 5, 1650-7, 1999
non-patent reference 6: Pathol. Oncol. Res. 4, 316-21, 1998
non-patent reference 7: Cancer Cell 3, 213-7, 2003

## Disclosure of the Invention

### Problems to be Solved by the Invention

In view of the current situation mentioned above, the present invention aims at providing an agent for the prophylaxis and/or treatment of a disease involving a heat shock protein.

### Means of Solving the Problems

The present inventors have conducted intensive studies in an attempt to solve the aforementioned problem and found that a certain kind of quinazoline derivative regulates the molecular chaperone function, and that the molecular chaperone function regulator is useful as a pharmaceutical composition for the prophylaxis and/or treatment of a disease involving a heat shock protein, or for a thermotherapy enhancing action, which resulted in the completion of the present invention.

Accordingly, the present invention provides the following.

The present invention relates to

(1) a molecular chaperone function regulator comprising, as an active ingredient, a quinazoline derivative represented by the following formula (I)

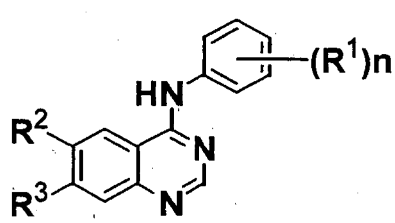

( I )

wherein n is an integer of 0 to 3, $R^1$ is a hydrogen atom, a halogen atom, hydroxy, cyano, nitro, trifluoromethyl, $C_{1-5}$ alkyl, $C_{1-5}$ alkoxy, $-S(O)_fR^{13}$ (wherein f is an integer of 0 to 2, and $R^{13}$ is $C_{1-5}$ alkyl), $-NR^{14}R^{15}$ (wherein $R^{14}$ and

$R^{15}$ are each independently a hydrogen atom, $C_{1-5}$ alkyl, $C_{1-5}$ alkanoyl or $C_{1-5}$ alkylsulfonyl), $C_{2-5}$ alkenyl, $C_{2-5}$ alkynyl or $C_{1-5}$ alkanoyl, one of $R^2$ and $R^3$ is
$R^{27}SO_2NH$- (wherein $R^{27}$ is $C_{1-5}$ alkyl optionally substituted by morpholino), $(R^{28}SO_2)_2N$- (wherein $R^{28}$ is $C_{1-5}$ alkyl optionally substituted by morpholino), $C_{1-5}$ alkoxy, $CH_3COCH_2CONH$-, $CH_3SCH_2CH_2CONH$-, $NCCH_2CONH$-,
**[0020]**

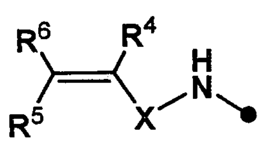

or

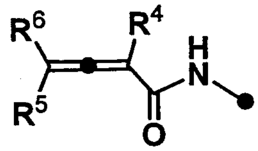

(wherein X is -C(O)- or $SO_2$-, and $R^4$, $R^5$ and $R^6$ are each independently a hydrogen atom, a halogen atom or $C_{1-5}$ alkyl optionally substituted by a halogen atom, morpholino, 4-$C_{1-5}$ alkylpiperazin-1-yl or di ($C_{1-5}$ alkyl)amino), or
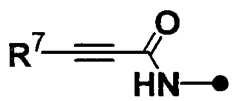

(wherein $R^7$ is $C_{1-5}$ alkyl optionally substituted by a hydrogen atom, a halogen atom, morpholino, 4-$C_{1-5}$ alkylpiperazin-1-yl or di ($C_{1-5}$ alkyl)amino), and
the other of $R^2$ and $R^3$ is
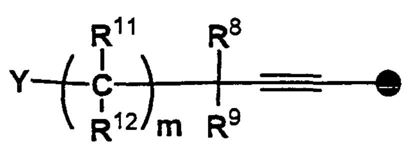

or

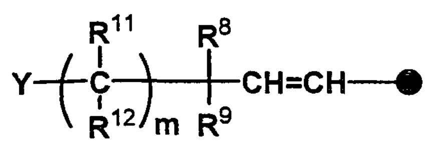

wherein $R^8$ and $R^9$ are each independently [a] a hydrogen atom, [b] $C_{1-5}$ alkyl optionally substituted by hydroxy or $C_{1-5}$ alkoxy, [c] $R^8$ and $R^9$ in combination show C=O, or [d] $R^8$ and $R^9$ in combination form a ring to show $C_{3-8}$ cycloalkylene optionally via -O-, -S- or -$NR^{10}$- (wherein $R^{10}$ is a hydrogen atom or $C_{1-5}$ alkyl), m is an integer of 0 to 3, $R^{11}$ and $R^{12}$ are each independently a hydrogen atom or $C_{1-5}$ alkyl, and Y is a hydrogen atom, hydroxy, $C_{1-5}$ alkoxy, $C_{1-5}$ alkanoyloxy, - $N(R^{16})$-(CO)u-$(CR^{17}R^{18})$v-(CO)j-$R^{19}$ (wherein $R^{16}$ is [a] a hydrogen atom or [b] $C_{1-5}$ alkyl optionally substituted by cyano or $C_{1-5}$ alkoxy, $R^{17}$ and $R^{18}$ are each independently a hydrogen atom or $C_{1-5}$ alkyl, u and j are each 0 or 1, v is an integer of 1 to 5, and $R^{19}$ is a hydrogen atom, hydroxy, cyano, amino, $C_{1-5}$ alkoxy, morpholino, 4-$C_{1-5}$ alkylpiperazin-1-yl or di($C_{1-5}$ alkyl) amino,
**[0026]** provided that when (i) u and j are simultaneously 0, then v is an integer of 2 to 5, and when (ii) $R^{19}$ is a cyano group, then j is 0),
**[0027]**

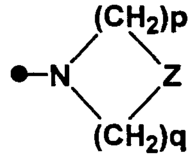

wherein p and q are each independently 2 or 3, and Z is -O-, - $S(O)_g$- (wherein g is an integer of 0 to 2), carbonyl or -$NR^{20}$-(wherein $R^{20}$ is [a] a hydrogen atom, [b] $C_{1-5}$ alkylsulfonyl, [c] $C_{1-5}$ alkanoyl, [d] $C_{1-5}$ alkoxycarbonyl, or [e] $C_{1-5}$ alkyl optionally substituted by cyano or $C_{1-5}$ alkoxy), or

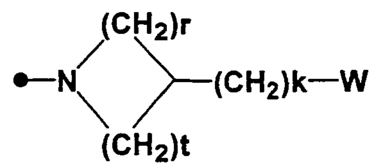

wherein r and t are each independently an integer of 1 to 3, k is 0 or 1, and W is a hydrogen atom, hydroxy, $C_{1-5}$ alkoxy, $C_{1-5}$ alkanoyloxy, carboxyl, cyano, di($C_{1-5}$ alkyl)amino, morpholino, pyrrolidin-1-yl, piperidin-1-yl, 4-$C_{1-5}$ alkylpiperazin-1-yl or $CONR^{21}R^{22}$ (wherein $R^{21}$ and $R^{22}$ are each independently a hydrogen atom or $C_{1-5}$ alkyl), or a pharmaceutically acceptable salt thereof, or a hydrate or solvate thereof, or an optically active form or racemate thereof or a diastereomer mixture thereof,

(2) the molecular chaperone function regulator of the aforementioned (1), wherein, in the quinazoline derivative of the aforementioned formula (I),
$R^2$ is $R^{27}SO_2NH$- (wherein $R^{27}$ is $C_{1-5}$ alkyl optionally substituted by morpholino), $(R^{28}SO_2)_2N$- (wherein $R^{28}$ is $C_{1-5}$ alkyl optionally substituted by morpholino), $C_{1-5}$ alkoxy, $CH_3COCH_2CONH$-, $CH_3SCH_2CH_2CONH$-, $N{\equiv}CCH_2CONH$-, or

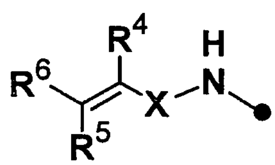

(wherein X is -C(O)- or $SO_2$- and $R^4$, $R^5$ and $R^6$ are each independently a hydrogen atom, a halogen atom or $C_{1-5}$ alkyl optionally substituted by a halogen atom, morpholino, 4-$C_{1-5}$ alkylpiperazin-1-yl or di($C_{1-5}$ alkyl)amino), and $R^3$ is

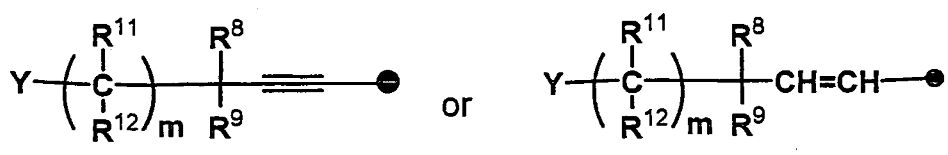

wherein m is an integer of 0 to 3,
$R^8$and $R^9$ are each independently a hydrogen atom, hydroxy or $C_{1-5}$ alkyl optionally substituted by $C_{1-5}$ alkoxy, $R^8$ and $R^9$ in combination show -C(O)-, or $R^8$ and $R^9$ in combination form a ring to show $C_{3-8}$ cycloalkylene optionally via -O-, -S- or - $NR^{10}$- (wherein $R^{10}$ is a hydrogen atom or $C_{1-5}$ alkyl), $R^{11}$ and $R^{12}$ are each independently a hydrogen atom or $C_{1-5}$ alkyl, and
Y is a hydrogen atom, hydroxy, $C_{1-5}$ alkoxy, $C_{1-5}$ alkanoyloxy, - $N(R^{16})$-(CO)u-$(CR^{17}R^{18})$v-(CO)j-$R^{19}$ (wherein $R^{16}$ is a hydrogen atom, or $C_{1-5}$ alkyl optionally substituted by cyano or $C_{1-5}$ alkoxy,
$R^{17}$ and $R^{18}$ are each independently a hydrogen atom or $C_{1-5}$ alkyl, u and j are each independently 0 or 1, v is an integer of 1 to 5, and
$R^{19}$ is a hydrogen atom, hydroxy, cyano, amino, $C_{1-5}$ alkoxy, morpholino, 4-$C_{1-5}$ alkylpiperazin-1-yl, or di ($C_{1-5}$ alkyl) amino,
**[0035]** provided that when (i) u and j are simultaneously 0, then v is an integer of 2 to 5, and when (ii) $R^{19}$ is cyano, then j is 0),

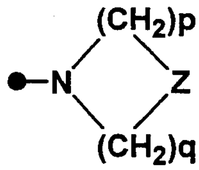

wherein p and q are each independently 2 or 3, and Z is -O-, - $S(O)_g$- (wherein g is an integer of 0 to 2), -C(O)- or $-NR^{20}$-(wherein $R^{20}$ is a hydrogen atom, $C_{1-5}$ alkylsulfonyl, $C_{1-5}$ alkanoyl, $C_{1-5}$ alkoxycarbonyl, or $C_{1-5}$ alkyl optionally substituted by cyano or $C_{1-5}$ alkoxy), or

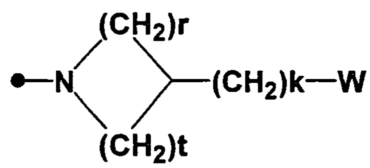

wherein r and t are each independently an integer of 1 to 3, k is 0 or 1, and W is a hydrogen atom, hydroxy, $C_{1-5}$ alkoxy, $C_{1-5}$ alkanoyloxy, carboxyl, cyano, di($C_{1-5}$ alkyl)amino, morpholino, pyrrolidin-1-yl, piperidin-1-yl, 4-$C_{1-5}$ alkylpiperazin-1-yl or $CONR^{21}R^{22}$ (wherein $R^{21}$ and $R^{22}$ are each independently a hydrogen atom or $C_{1-5}$ alkyl),

(3) the molecular chaperone function regulator of the aforementioned (1) or (2), wherein, in the quinazoline derivative of the aforementioned formula (I),

$R^3$ is

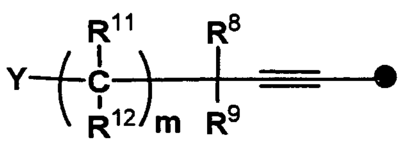

or

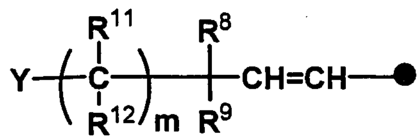

wherein m is an integer of 0 to 3,
$R^8$ and $R^9$ are each independently a hydrogen atom or $C_{1-5}$ alkyl optionally substituted by $C_{1-5}$ alkoxy, or $R^8$ and $R^9$ in combination form a ring to show $C_{3-8}$ cycloalkylene optionally via -O- or -NH-,
$R^{11}$ and $R^{12}$ are each independently a hydrogen atom or $C_{1-5}$ alkyl, and
Y is a hydrogen atom, hydroxy, $C_{1-5}$ alkoxy, $C_{1-5}$ alkanoyloxy, - $N(R^{16})$-(CO)u-$(CR^{17}R^{18})$v-(CO)j-$R^{19}$ (wherein $R^{16}$ is a hydrogen atom, or $C_{1-5}$ alkyl optionally substituted by cyano or $C_{1-5}$ alkoxy,
$R^{17}$ and $R^{18}$ are each independently a hydrogen atom or $C_{1-5}$ alkyl, u and j are each independently 0 or 1, v is an integer of 1 to 5, and $R^{19}$ is a hydrogen atom, hydroxy, cyano, amino, $C_{1-5}$ alkoxy, morpholino, 4-$C_{1-5}$ alkylpiperazin-1-yl, or di($C_{1-5}$ alkyl)amino, provided that when (i) u and j are simultaneously 0, then v is an integer of 2 to 5, and when (ii) $R^{19}$ is cyano,
then j is 0),

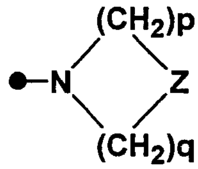

wherein p and q are each independently 2 or 3, and Z is -O-, - C(O)- or $-NR^{20}-$ (wherein $R^{20}$ is a hydrogen atom, $C_{1-5}$ alkylsulfonyl, $C_{1-5}$ alkanoyl, $C_{1-5}$ alkoxycarbonyl, or $C_{1-5}$ alkyl optionally substituted by cyano or $C_{1-5}$ alkoxy), or
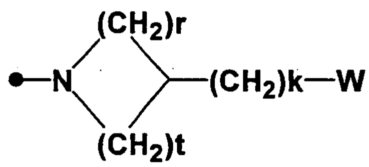

wherein r and t are each independently an integer of 1 to 3, k is 0 or 1, and W is a hydrogen atom, hydroxy, $C_{1-5}$ alkoxy, $C_{1-5}$ alkanoyloxy, carboxyl, cyano, di($C_{1-5}$ alkyl)amino, morpholino or $CONR^{21}R^{22}$ (wherein $R^{21}$ and $R^{22}$ are each independently a hydrogen atom or $C_{1-5}$ alkyl),

(4) the molecular chaperone function regulator of any one of the aforementioned (1) to (3), wherein, in the quinazoline derivative of the aforementioned formula (I),

$R^3$ is
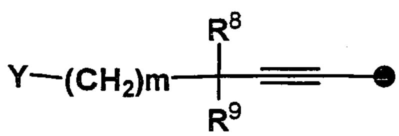

or

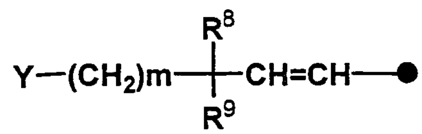

wherein m is an integer of 0 or 1,
$R^8$ and $R^9$ are each independently a hydrogen atom or $C_{1-5}$ alkyl optionally substituted by $C_{1-5}$ alkoxy, or $R^8$ and $R^9$ in combination form a ring to show $C_{3-8}$ cycloalkylene optionally via -O- or -NH-, and
Y is $C_{1-5}$ alkoxy, $-N(R^{16})-(CO)u-(CH_2)_2-(CO)j-R^{19}$ (wherein u and j are each 0 or 1, $R^{16}$ is a hydrogen atom, or $C_{1-5}$ alkyl optionally substituted by $C_{1-5}$ alkoxy, and $R^{19}$ is a hydrogen atom, cyano, $C_{1-5}$ alkoxy, morpholino, 4-$C_{1-5}$ alkylpiperazin-1-yl or di($C_{1-5}$ alkyl) amino, provided that when $R^{19}$ is cyano, then j is 0),
**[0048]**

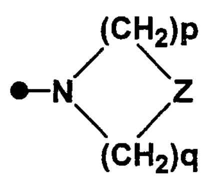

wherein p and q are each independently 2 or 3, and Z is -O- or $-NR^{20}-$ (wherein $R^{20}$ is a hydrogen atom, $C_{1-5}$ alkylsulfonyl, $C_{1-5}$ alkanoyl, $C_{1-5}$ alkoxycarbonyl, or $C_{1-5}$ alkyl optionally substituted by cyano or $C_{1-5}$ alkoxy), or
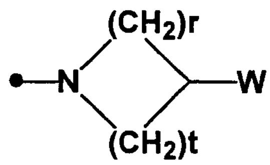

wherein r and t are each independently 1 or 2, and
W is a hydrogen atom, hydroxy, $C_{1-5}$ alkoxy, $C_{1-5}$ alkanoyloxy or carboxyl,

(5) the molecular chaperone function regulator of any one of the aforementioned (1) to (4), wherein, in the quinazoline derivative of the aforementioned formula (I),

$R^3$ is
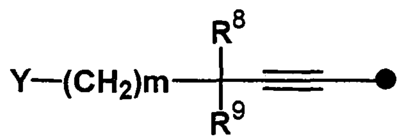

wherein m is 0 or 1,
$R^8$ and $R^9$ are each independently a hydrogen atom or $C_{1-5}$ alkyl optionally substituted by $C_{1-5}$ alkoxy, or $R^8$ and $R^9$ in combination form a ring to show $C_{3-8}$ cycloalkylene optionally via -O- or -NH-, and
Y is $C_{1-5}$ alkoxy, $-N(R^{16})-(CH_2)_2-R^{19}$ (wherein $R^{16}$ is a hydrogen atom or $C_{1-5}$ alkyl optionally substituted by $C_{1-5}$ alkoxy, and $R^{19}$ is a hydrogen atom, cyano, $C_{1-5}$ alkoxy, morpholino, 4-$C_{1-5}$ alkylpiperazin-1-yl, or di($C_{1-5}$ alkyl)amino, provided that when $R^{19}$ is cyano, then j is 0),
**[0054]**

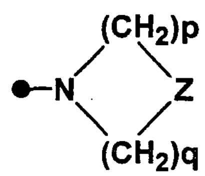

wherein p and q are each independently 2 or 3, and
Z is -O- or $-NR^{20}-$ (wherein $R^{20}$ is a hydrogen atom, $C_{1-5}$ alkylsulfonyl, $C_{1-5}$ alkanoyl, $C_{1-5}$ alkoxycarbonyl, or $C_{1-5}$ alkyl optionally substituted by cyano or $C_{1-5}$ alkoxy), or
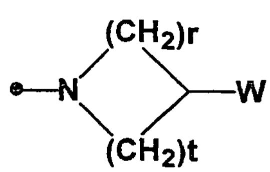

wherein r and t are each independently 1 or 2, and
W is a hydrogen atom, hydroxy, $C_{1-5}$ alkoxy, $C_{1-5}$ alkanoyloxy, carboxyl,

(6) the molecular chaperone function regulator of any one of the aforementioned (1) to (5), wherein, in the quinazoline derivative of the aforementioned formula (I),

$R^2$ is $R^{27}SO_2NH-$ (wherein $R^{27}$ is $C_{1-5}$ alkyl), $C_{1-5}$ alkoxy or

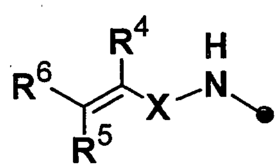

wherein each symbol is as defined above,

(7) the molecular chaperone function regulator of any one of the aforementioned (1) to (6), wherein, in the quinazoline derivative of the aforementioned formula (I), n is 1 or 2, and $R^1$ is a halogen atom, cyano, $C_{1-5}$ alkyl, $C_{1-5}$ alkoxy, $-NR^{14}R^{15}$ (wherein $R^{14}$ and $R^{15}$ are each independently a hydrogen atom, $C_{1-5}$ alkyl, $C_{1-5}$ alkanoyl or $C_{1-5}$ alkylsulfonyl), $C_{2-5}$ alkynyl, or $C_{1-5}$ alkanoyl,

(8) the molecular chaperone function regulator of any one of the aforementioned (1) to (7), wherein, in the quinazoline derivative of the aforementioned formula (I), n is 2, $R^1$ is a halogen atom, $R^2$ is

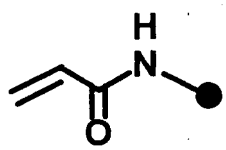

,

and

$R^3$ is

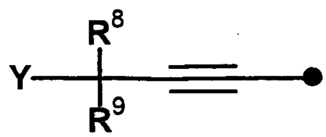

wherein $R^8$ and $R^9$ are each $C_{1-5}$ alkyl optionally substituted by $C_{1-5}$ alkoxy, and Y is $-N(R^{16})-(CH_2)_2-R^{19}$ (wherein $R^{16}$ is $C_{1-5}$ alkyl optionally substituted by $C_{1-5}$ alkoxy, and $R^{19}$ is di($C_{1-5}$ alkyl)amino), or

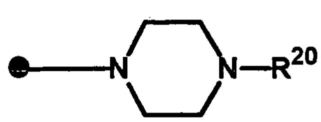

wherein $R^{20}$ is $C_{1-5}$ alkyl optionally substituted by $C_{1-5}$ alkoxy,

(9) the molecular chaperone function regulator of any one of the aforementioned (1) to (8), wherein the quinazoline derivative is N-{4-[(3-chloro-4-fluorophenyl)amino]-7-[3-methyl-3-(4-methyl-1-piperazinyl)-1-butynyl]-6-quinazolinyl}acrylamide bis(4-methylbenzenesulfonate),

(10) a pharmaceutical composition for the prophylaxis and/or treatment of a disease involving a heat shock protein, or for a thermotherapy enhancing action, which comprises the molecular chaperone function regulator of any one of the aforementioned (1) to (9),

(11) the pharmaceutical composition of the aforementioned (10), wherein the disease involving a heat shock protein

is a hormone-dependent disease, a hematopoietic tumor or a malignant soft tissue tumor,

(12) the pharmaceutical composition of the aforementioned (11), wherein the hormone-dependent disease is prostatic hyperplasia, endometriosis or a hormone-sensitive cancer,

(13) the pharmaceutical composition of the aforementioned (12), wherein the hormone-sensitive cancer is a hormone-sensitive breast cancer, a hormone-sensitive prostate cancer or a hormone-sensitive endometrial cancer,

(14) the pharmaceutical composition of the aforementioned (11), wherein the hematopoietic tumor is leukemia, malignant lymphoma or myeloma,

(15) the pharmaceutical composition of the aforementioned (11), wherein the malignant soft tissue tumor is osteosarcoma or soft tissue sarcoma,

(16) a pharmaceutical composition for the prophylaxis and/or treatment of a disease involving a heat shock protein, or for a thermotherapy enhancing action, which comprises a quinazoline derivative represented by the following formula (I)

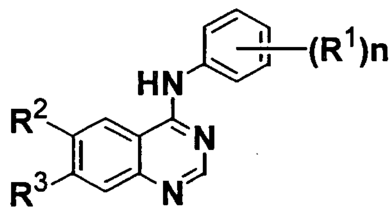

( I )

wherein each symbol is as defined in the aforementioned (1), or a pharmaceutically acceptable salt thereof, a hydrate or solvate thereof, an optically active form or racemate thereof or a diastereomer mixture thereof,

(17) the pharmaceutical composition of the aforementioned (16), wherein the disease involving a heat shock protein is a hormone-dependent disease, a hematopoietic tumor, or a malignant soft tissue tumor,

(18) the pharmaceutical composition of the aforementioned (17), wherein the hormone-dependent disease is prostatic hyperplasia, endometriosis or a hormone-sensitive cancer,

(19) the pharmaceutical composition of the aforementioned (18), wherein the hormone-sensitive cancer is a hormone-sensitive breast cancer, a hormone-sensitive prostate cancer or a hormone-sensitive endometrial cancer,

(20) the pharmaceutical composition of the aforementioned (17), wherein the hematopoietic tumor is leukemia, malignant lymphoma or myeloma, and

(21) the pharmaceutical composition of the aforementioned (17), wherein the malignant soft tissue tumor is osteosarcoma or soft tissue sarcoma.

## Effect of the Invention

The present invention can provide a molecular chaperone function regulator comprising, as an active ingredient, a quinazoline derivative represented by the aforementioned formula (I) or a pharmaceutically acceptable salt thereof, or a hydrate or solvate thereof, or an optically active form or racemate thereof or a diastereomer mixture thereof.

## Brief Description of the Drawings

Fig. 1 is a graph showing the time-course changes of HSP70 induction in human epithelial cancer-derived A-431 cell, wherein the vertical axis shows the expression amount of HSP70 in the cell treated with a compound to the expression amount of HSP70 in the cell in the control well as 100, and the horizontal axis shows treatment time.

Fig. 2 shows expression of HSP70 in various tumor cells as assayed by the Western blot, wherein each lane shows C: DMSO, A: compound A, and ZD: gefinitib.

Fig. 3 shows expression of client proteins in human breast cancer-derived MCF-7 cells as assayed by the Western blot, wherein each lane shows C: DMSO, GA: geldanamycin, A: compound A, and ZD: gefinitib, as for ERα and AR, nuclear fractions were treated with respective antibodies, and as for HER2, AKT and CDK4, cell extracts were treated with respective antibodies.

Fig. 4 shows an effect of a compound on cell growth upon stimulation of human breast cancer-derived MCF-7 cells

with estradiol, wherein the vertical axis shows suppression rate, the horizontal axis shows concentrations of the compound, A is compound A and TAM is tamoxifen.

Fig. 5 shows an effect of hyperthermic treatment and compound A on the growth of human prostate cancer cell-derived DU 145 cells, wherein the vertical axis shows absorbance of a compound-treated well or a heat-treated well to the absorbance of a control well of a plate free of the heat treatment as 100, and the horizontal axis shows concentrations of compound A.

Fig. 6 shows an effect of compound A on the growth of human breast cancer MCF-7 implanted into nude mouse, wherein the vertical axis shows tumor weights and the horizontal axis shows doses of compound A. 5 per group, *: $p<0.05$, by Dunnett's test with a solvent administration group as a control.

Fig. 7 shows an effect of compound A on ubiquitination of protein in human breast cancer-derived T-47D cell, wherein the vertical axis shows the rate of ubiquitinated protein treated with a compound to the control as 100 and the horizontal axis shows treatment time.

**Best Mode for Embodying the Invention**

Respective definitions in the present invention are as follows.

The compound of the present invention is a quinazoline derivative represented by the aforementioned formula (I).

As the halogen atom defined for each substituent of the aforementioned formula (I), fluorine atom, chlorine atom, bromine atom and iodine atom can be mentioned; as the $C_1$-$C_5$ alkyl group, methyl group, ethyl group, n-propyl group, isopropyl group, n-butyl group, iso-butyl group, sec-butyl group, tert-butyl group, n-pentyl group, neopentyl group and the like can be mentioned; as the $C_1$-$C_5$ alkoxy group, methoxy group, ethoxy group, n-propoxy group, iso-propoxy group, n-butoxy group, iso-butoxy group, sec-butoxy group, tert-butoxy group, n-pentyloxy group, neopentyloxy group and the like can be mentioned; as the $C_2$-$C_5$ alkenyl group, vinyl group, 1-propenyl group, 2-propenyl group, 1-butenyl group, 2-methylpropen-1-yl group, 2-butenyl group, 1-pentenyl group, 2-pentenyl group and the like can be mentioned; as the $C_2$-$C_5$ alkynyl group, ethynyl group, 1-propynyl group, 1-butynyl group, 1-pentynyl group and the like can be mentioned; as the $C_1$-$C_5$ alkanoyl group, formyl group, acetyl group, propionyl group, butyryl group, isovaleryl group, valeryl group and the like can be mentioned; as the $C_1$-$C_5$ alkylsulfonyl group, methylsulfonyl group, ethylsulfonyl group and the like can be mentioned; as the $C_3$-$C_5$ cycloalkylene group, cyclopropane group, cyclobutane group, cyclopentane group, cyclohexane group and the like can be mentioned, as the $C_1$-$C_5$ alkanoyloxy group, acetyloxy group, propionyloxy group and the like can be mentioned, as the $C_1$-$C_5$ alkylsulfonyl group, methylsulfonyl group, ethylsulfonyl group and the like can be mentioned, and as the $C_1$-$C_5$ alkoxycarbonyl group, methoxycarbonyl group, ethoxycarbonyl group and the like can be mentioned.

The quinazoline derivative of the present invention is converted to a salt with the corresponding acid or base by a known method.

Examples of the salt include inorganic acid salts such as hydrochloride, sulfate, carbonate, phosphate and the like, and organic acid salts such as formate, acetate, propionate, lactate, oxalate, fumarate, maleate, citrate, tartrate, benzoate, phthalate, methanesulfonate, p-toluenesulfonate, isethionate, glucuronate, gluconate and the like. In addition, alkali metal salts such as sodium salt, potassium salt and the like, alkaline earth metal salts such as magnesium salt, calcium salt and the like, ammonium salt, a salt with a pharmacologically acceptable organic amine (tetramethylamine, triethylamine, benzylamine, phenethylamine, monoethanolamine, diethanolamine, tris(hydroxyethylamine), lysine and arginine etc.) can be mentioned.

The quinazoline derivative of the present invention can have various steric structures. For example, when considered from an asymmetric carbon atom as a center, the absolute configuration thereof may be (S)-form or (R)-form, or a racemate. Pure forms of optical isomer and diastereoisomer, optional mixtures of the isomers, racemate and the like are all encompassed in the present invention.

The quinazoline derivative of the formula (I) can be present in the form of, for example, a solvate such as hydrate or a non-solvate, and the present invention encompasses all such kinds of solvates having an anticancer activity.

Preferable embodiments of the compounds of the present invention are shown in the following Tables 1-9. In the Tables, Me means methyl group, Et means ethyl group and Pr means propyl group.

Table 1

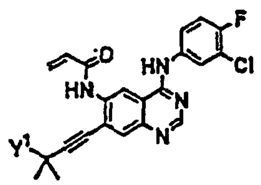

| Y[1] | Y[1] | Y[1] |
|---|---|---|
| 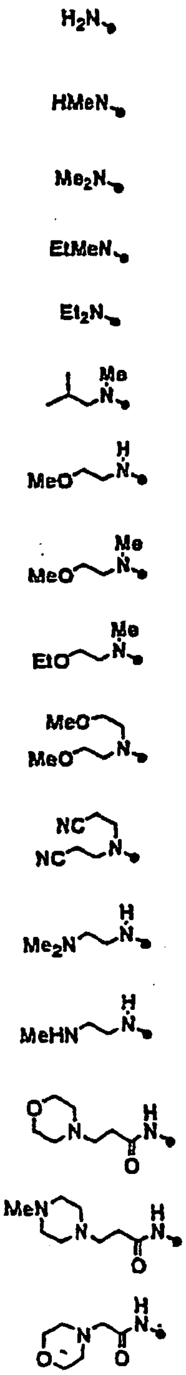 | 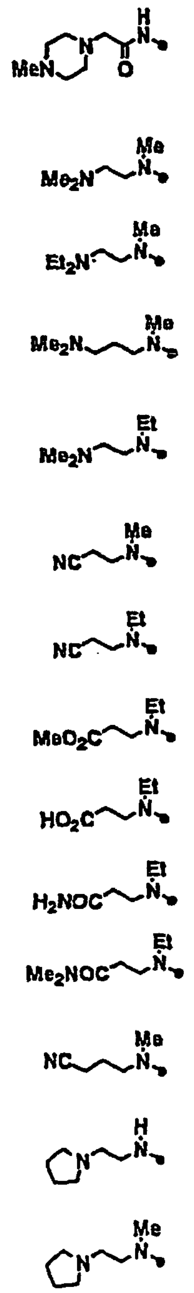 | 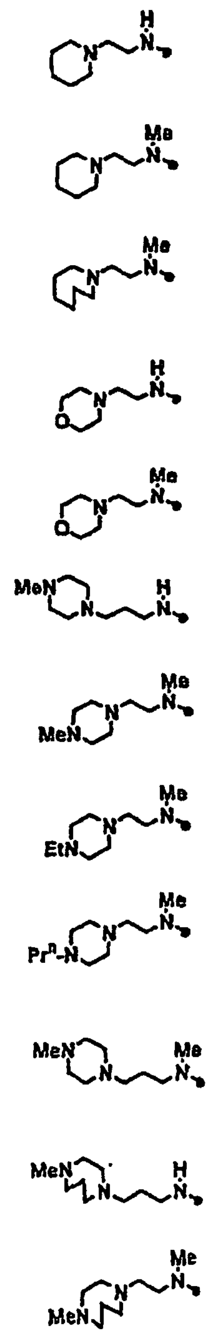 |

Table 2

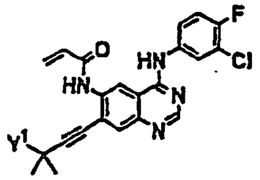

| $Y^1$ | $Y^1$ | $Y^1$ |
|---|---|---|
| 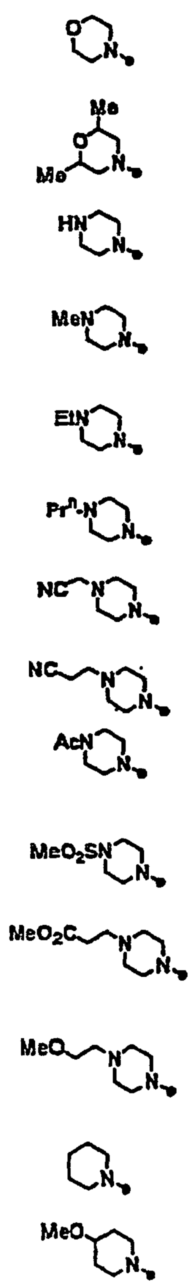 | 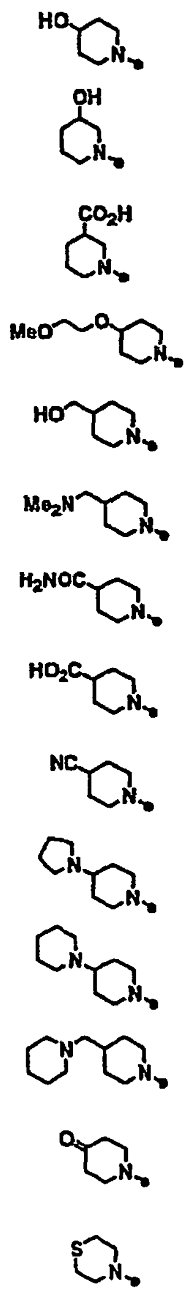 | 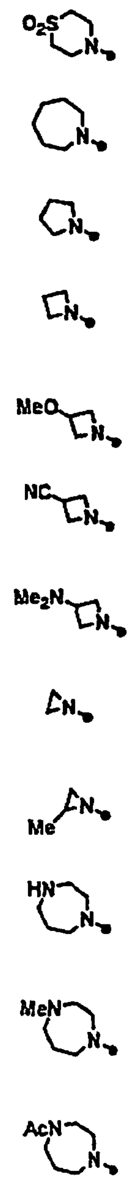 |

Table 3

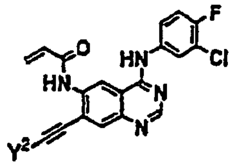

| $Y^2$ | $Y^2$ |
|---|---|
| 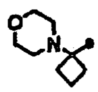 | 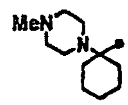 |
| 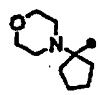 | 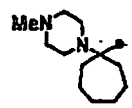 |
| 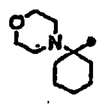 | 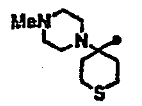 |
| 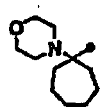 | 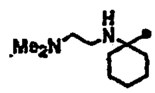 |
| 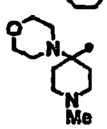 | 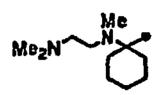 |
| 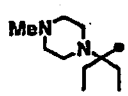 | 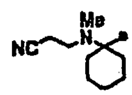 |
| 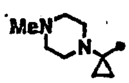 | 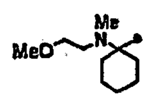 |
| 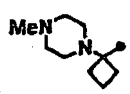 | 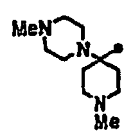 |
| 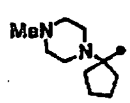 | 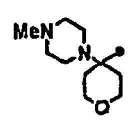 |
| 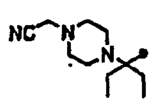 | |
| 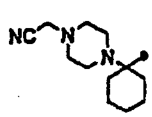 | |

Table 4

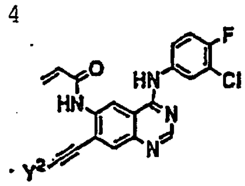

| $Y^2$ | $Y^2$ | $Y^2$ |
|---|---|---|
| 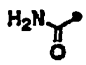 | 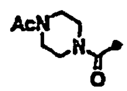 | 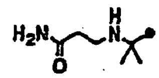 |
| 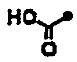 | 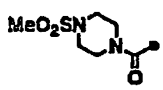 | 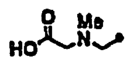 |
| 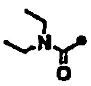 | 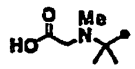 | 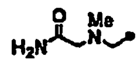 |
| 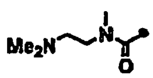 | 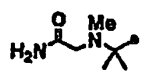 | 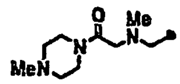 |
| 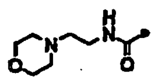 | 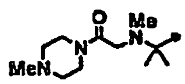 | 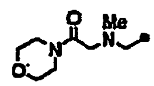 |
| 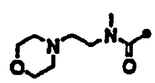 | 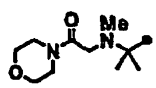 | 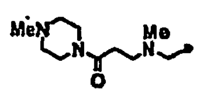 |
| 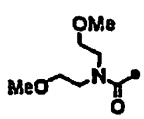 | 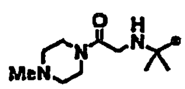 | 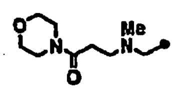 |
| 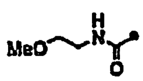 | 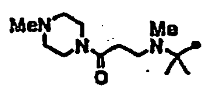 | 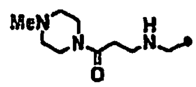 |
| 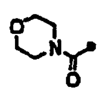 | 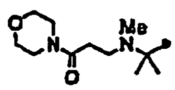 | 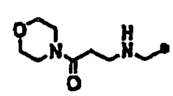 |
| 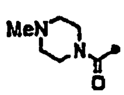 | 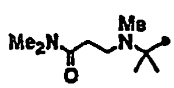 | 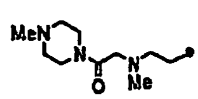 |
| 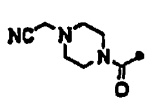 | 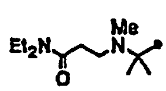 | 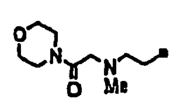 |
| 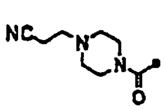 | 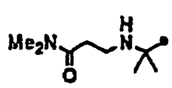 | 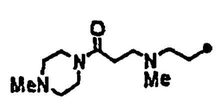 |

Table 5

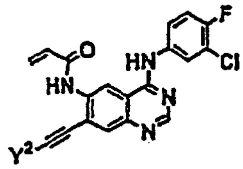

| $Y^2$ | $Y^2$ |
|---|---|
| 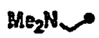 | 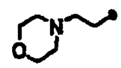 |
| 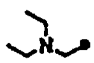 | 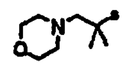 |
| 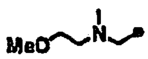 | 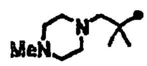 |
| 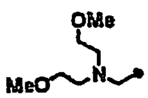 | 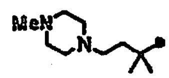 |
| 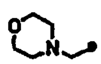 | 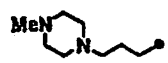 |
| 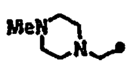 | 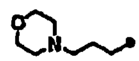 |
| 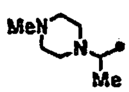 | 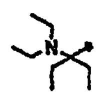 |
| 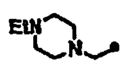 | 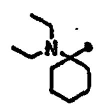 |
| 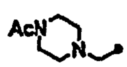 | 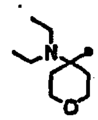 |
| 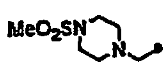 | 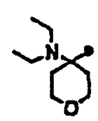 |
| 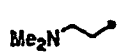 | 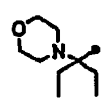 |
| 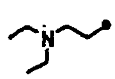 | 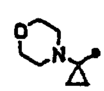 |
| 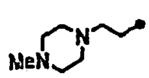 | |

Table 6

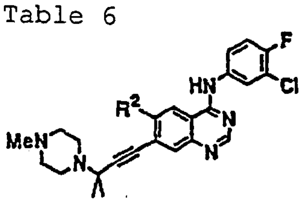

| $R^2$ | $R^2$ | $R^2$ |
|---|---|---|
| 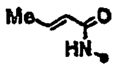 | 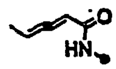 | 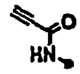 |
| 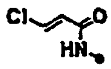 | 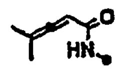 | 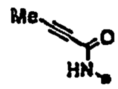 |
| 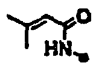 | 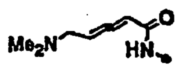 | 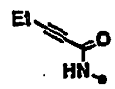 |
| 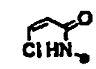 | 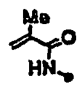 | 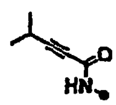 |
| 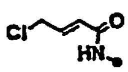 | 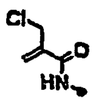 | 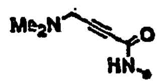 |
| 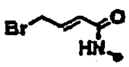 | 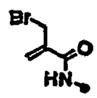 | 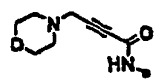 |
| 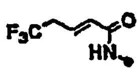 | 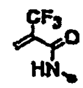 | 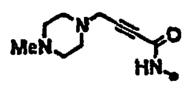 |
| 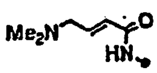 | 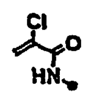 | 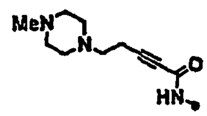 |
| 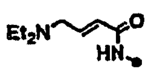 | 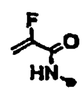 | 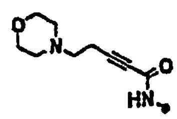 |
| 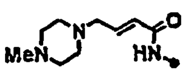 | 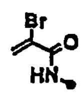 | 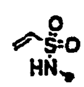 |
| 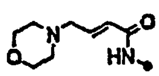 | 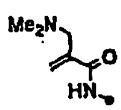 | 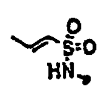 |
| 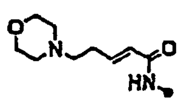 | 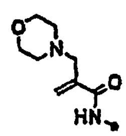 | |
| 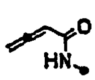 | | |

Table 7

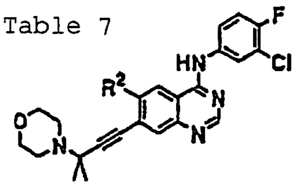

| $R^2$ | $R^2$ | $R^2$ |
|---|---|---|
| 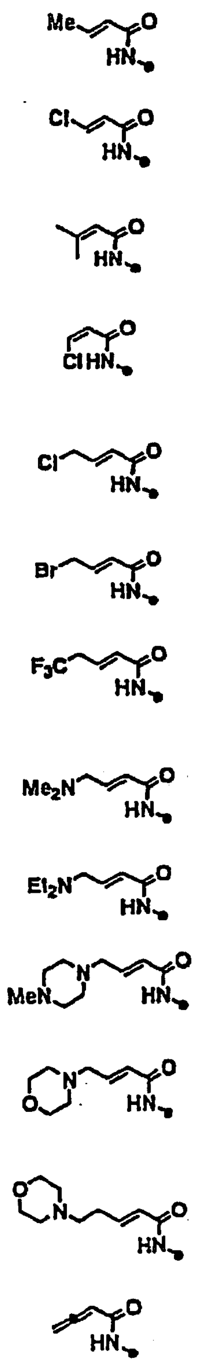 | 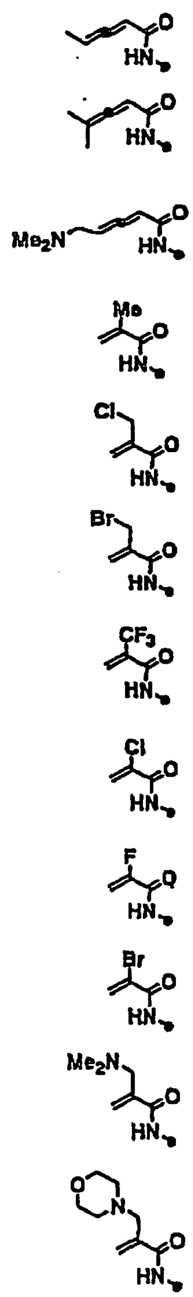 | 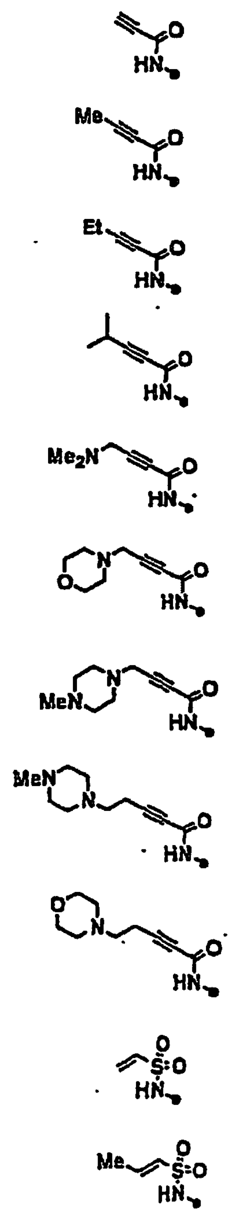 |

Table 8

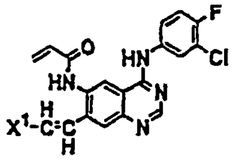

| $X^1$ | $X^1$ | $X^1$ |
|---|---|---|
| 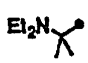 | 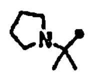 | 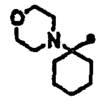 |
| 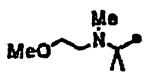 | 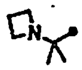 | 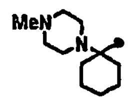 |
| 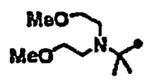 | 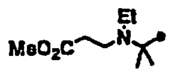 | 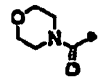 |
| 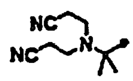 | 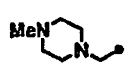 | 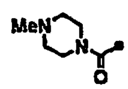 |
| 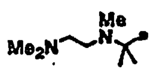 | 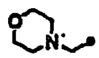 | 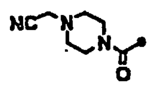 |
| 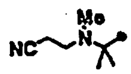 | 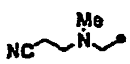 | 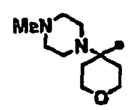 |
| 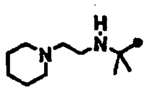 | 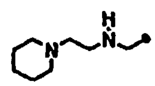 | 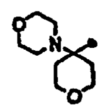 |
| 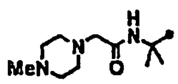 | 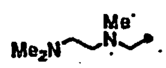 | 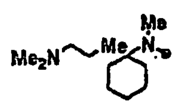 |
| 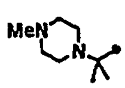 | 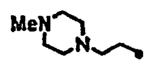 | 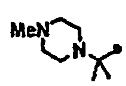 |
| 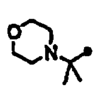 | 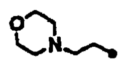 | 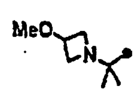 |
| 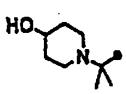 | 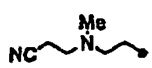 | 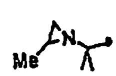 |
| 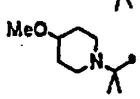 | 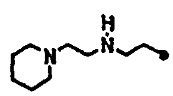 | |
| 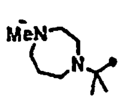 | 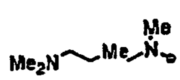 | |

Table 9

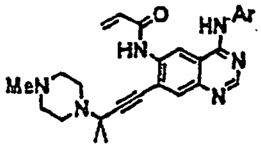

| Ar | Ar | Ar |
|---|---|---|
| 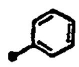 | 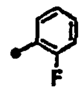 | 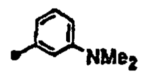 |
| 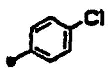 | 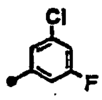 | 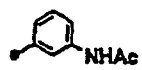 |
| 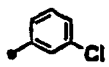 | 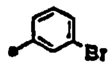 | 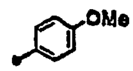 |
| 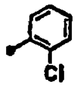 | 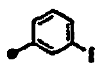 | 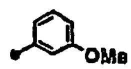 |
| 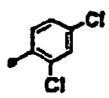 | 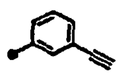 | 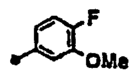 |
| 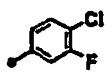 | 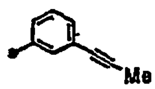 | 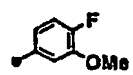 |
| 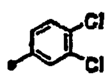 | 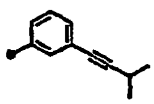 | 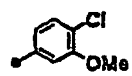 |
| 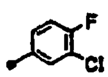 | 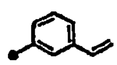 | 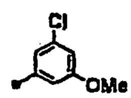 |
| 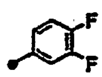 | 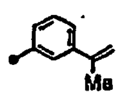 | 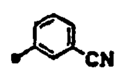 |
| 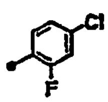 | 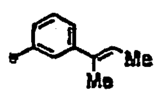 | 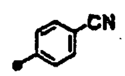 |
| 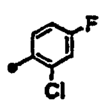 | 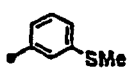 | 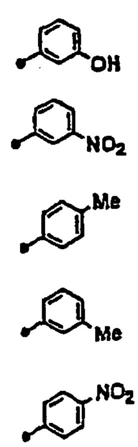 |
| 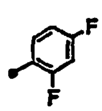 | 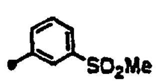 | |
| 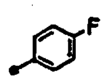 | 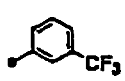 | |
| 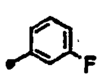 | 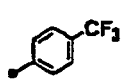 | |
| | | |

In the present invention, the "heat shock protein" may be any suitable heat shock protein (HSP) or a complex thereof, and is preferably HSP70 and HSP90.

In the present invention, the "regulation of molecular chaperone function" means promotion of inhibition of the molecular chaperone function and degradation of client proteins through regulation of expression amount of HSP itself or action on the formation of a HSP complex including client proteins.

In the present invention, specific examples of the "disease involving a heat shock protein" include hormone-dependent diseases, hematopoietic tumors and malignant soft tissue tumors.

In the present invention, the "hormone-dependent disease" is a disease requiring hormone for the pathology activity of the disease, and is a suitable disease wherein steroid hormone is involved in the pathology activity. Specifically, prostatic hyperplasia, endometriosis, hormone-sensitive cancer and the like can be mentioned.

In the present invention, specific examples of the "hormone-sensitive cancer" include hormone-sensitive breast cancer, hormone-sensitive prostate cancer and hormone-sensitive endometrial cancer.

Examples of the aforementioned compound represented by the formula (I) in the present specification include quinazoline compounds described in WO02/66445, and the production method of the compounds is described in the above-mentioned specification.

Moreover, a pharmaceutically acceptable salt of the aforementioned formula (I), a hydrate thereof, a solvate thereof, an optically active form or racemate thereof and a diastereomer mixture thereof can be synthesized by a conventional method.

The aforementioned compound of the formula (I) obtained by the above-mentioned method, a pharmaceutically acceptable salt thereof, a hydrate or solvate thereof, an optically active form or racemate thereof and a diastereomer mixture thereof are useful as molecular chaperone function regulators, useful as drugs for the prophylaxis and/or treatment of a disease involving a heat shock protein, and further useful as thermotherapy enhancing drugs.

While one or more kinds of the aforementioned compound of the formula (I) or a salt thereof, which is the active ingredient, may be directly administered to patients as the prophylactic and/or therapeutic agent of the present invention, preferably, a pharmacologically and pharmaceutically acceptable additive for the active ingredient is added, and the mixture should be provided in the form of a preparation well known to those of ordinary skill in the art.

As the pharmacologically and pharmaceutically acceptable additive, for example, excipient, disintegrant or disintegration aids, binder, lubricant, coating agent, dye, diluent, base, dissolving agent and dissolution aids, isotonicity agent, pH regulator, stabilizer, spray, adhesive and the like can be used. Examples of the preparation suitable for oral administration include tablet, capsule, powder, fine granule, granule, liquid, syrup and the like and examples of the preparation suitable for parenteral administration include injection, drip infusion, suppository and the like.

The preparation suitable for oral administration can contain excipient, disintegrant, disintegration aids, binder, lubricant, coating agent, base and the like as additives.

The preparation suitable for injection or infusion can contain additives for preparation, such as dissolving agent, dissolution aids, isotonicity agent, pH regulator and the like.

Moreover, the molecular chaperone function regulator of the present invention can be used in combination with an anticancer agent such as 5-fluorouracil, gemcitabine, doxorubicin, irinotecan, cisplatin, paclitaxel, vincristine, etoposide, trastuzumab, imatinib and the like, a hormonal therapeutic agent such as leuprorelin, tamoxifen, anastrozole, goserelin and the like, an antiemetic agent such as granisetron, dexamethasone, metoclopramide and the like, and the like.

The administration route of the pharmaceutical agent of the present invention is not particularly limited, and the agent can be orally or parenterally administered. For the disease involving HSP, for example, intravenous administration, intraarterial administration or intracardiac injection for the purpose of preventing aggravation of the condition, ameliorating the symptoms and the like is preferable.

While the dose of the pharmaceutical agent of the present invention can be appropriately determined according to the desired prophylaxis and/or treatment of the disease involving heat shock proteins, conditions such as age and state of patients and the like, generally, about 0.001 - 100 mg/kg is preferably administered to an adult by injection or infusion once a day or in two or more divided portions, or about 0.001 - 100 mg/kg is preferably administered orally to an adult once a day or in two or more divided portions.

## Examples

The present invention is explained in detail in the following by referring to Examples, which are not to be construed as limitative as long as they do not go beyond the gist of the invention.

The reagents, compounds and tumor cells used for the experiment of each Example were as follows.

HSP70 specific antibody: manufactured by StressGen Biotechnologies Corp., #SPA-810
anti-HER2 antibody: manufactured by NeoMarkers Inc., Ab-15

anti-EGFR antibody: manufactured by Upstate Inc., #06-129
anti-ERα antibody: manufactured by Santa Cruz Biotechnology, Inc., sc-7207
anti-AR antibody: manufactured by Santa Cruz Biotechnology, Inc., sc-815
anti-AKT antibody: manufactured by Cell Signaling Technology, Inc., #9272
anti-CDK4 antibody: manufactured by Upstate Inc., #06-139
anti-ubiquitin antibody: manufactured by Santa Cruz Biotechnology, Inc., sc-8017
compound A: The following compounds were synthesized according to the description of WO02/66445 and used for the experiment. N-{4-[(3-Chloro-4-fluorophenyl)amino]-7-[3-methyl-3-(4-methyl-1-piperazinyl)-1-buthynyl]-6-quinazolinyl}acrylamide bis(4-methylbenzenesulfonate)}

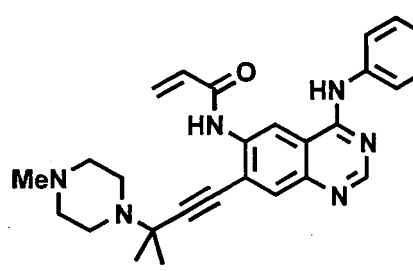

2TsOH,n$H_2O$

Compounds B - F: The following compounds were synthesized according to the description of WO02/66445 and used for the experiment.

Compound B: N-([4-(3-Chloro-4-fluorophenyl)amino]-7-{3-[4-(2-methoxyethyl)-1-piperazinyl]-3-methyl-1-butynyl}-6-quinazolinyl)acrylamide

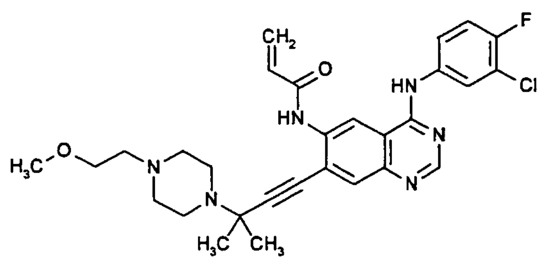

Compound C: N-{4-[(3-Chloro-4-fluorophenyl)amino]-7-[3-(4-methyl-1-piperazinyl)-1-propynyl]-6-quinazolinyl} acrylamide

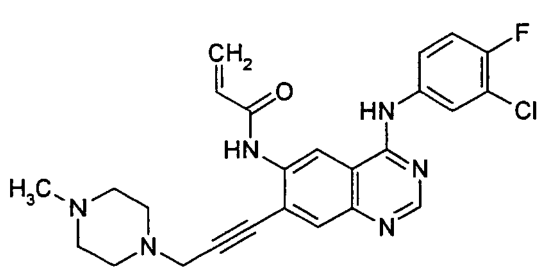

Compound D: N-{4-[(3-Cyano-phenyl)amino]-7-[3-methyl-3-(4-methyl-1-piperazinyl)-1-butynyl]-6-quinazolinyl}acrylamide

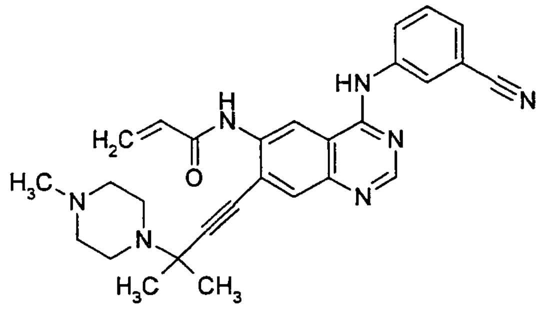

Compound E: N-{4-[(3-Chloro-4-fluorophenyl)amino]-7-[4-(4-methyl-1-piperazinyl)-4-tetrahydropyranylethynyl]-6-quinazolinyl}acrylamide

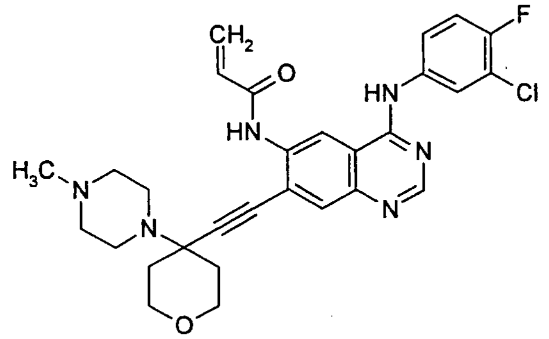

Compound F: (3-Chloro-4-fluorophenyl)-(7-ethoxy-6-{3-methyl-3-[methyl-(1-methyl-4-piperidinyl)amino]-1-butynyl}-4-quinazolinyl)amine

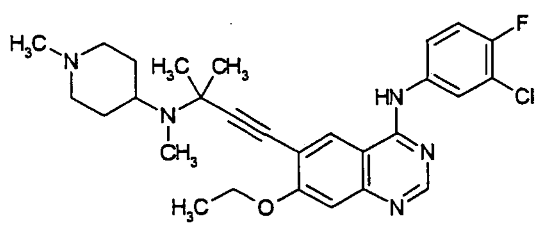

gefinitib: The following compound having a quinazoline skeleton and known as an anti-malignant tumor agent was synthesized according to the description of WO96/33980 and used for the experiment as a control drug. 4-(3'-Chloro-4'-fluoroanilino)-7-methoxy-6-(3-morpholinopropoxy)quinazoline

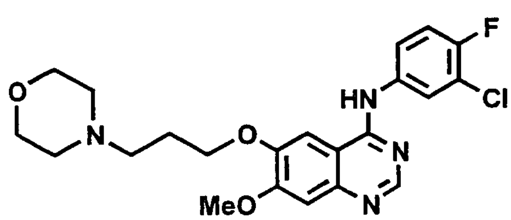

geldanamycin: Cat. # G 3381, obtained from Sigma Ltd. bortezomib: The following compound known as a proteasome inhibitor was synthesized according to the description of US Patent No. 6083903 and used for the experiment as a control drug.

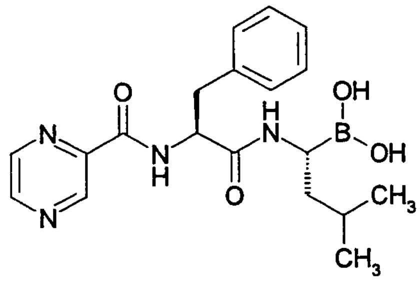

XTT (2,3-bis(2-methoxy-4-nitro-5-sulfophenyl)-2H-tetrazolium-5-carboxanilide) was used as a reagent: Cat. # X 4251, obtained from Sigma Ltd.

A-431 cell: human epithelial cancer-derived A-431 cell, obtained from Cell Resource Center for Biomedical Research Institute of Development, Aging and Cancer, Tohoku University School of Medicine (hereinafter indicated as Cell Resource Center).
TE-8 cell: human esophagus cancer-derived TE-8 cell (obtained from Cell Resource Center)
NCI-H520 cell: human lung cancer-derived NCI-H520 cell (obtained from ATCC)
HPAC cell: human pancreas cancer-derived HPAC cell (obtained from ATCC)
DU145 cell: human prostate cancer-derived DU145 cell (obtained from ATCC)
KPL-4 cell: human breast cancer-derived KPL-4 cell (provided by Mr. KUREBAYASHI Junichi, Kawasaki Medical School,
reference: Kurebayashi, J., et al., British J Cancer, 79, 707-717, 1999)
MKN-45 cell: human stomach cancer-derived MKN-45 cell (obtained from Health Science Research Resources Band)
DLD-1 cell: human colon cancer-derived DLD-1 cell (obtained from ATCC)
MCF-7 cell: human breast cancer-derived MCF-7 cell (obtained from ATCC)
T-47D cell: human breast cancer-derived T-47D cell (obtained from ATCC)

**Example 1** Time-dependent HSP70 induction using human epithelial cancer-derived A-431 cell

(Method) A-431 cells were cultured in a 12-well plate and, at the time point when the cells grew to about 70-80%, compound A and a control drug gefinitib were each added to 10 $\mu$M.

DMSO (0.1%) was added to the control. After the addition, the cells were collected 6, 9, 12 and 24 hr later and cell extracts were prepared. Expression of HSP70 protein in the cell extracts was examined by Western blot using an HSP70 specific antibody. T/C (%) was determined using the amount of chemical luminescence on a PVDF film due to an HRP-labeled secondary antibody as the HSP70 expression amount and HSP70 expression amount of the cells in the control well as 100.

The results are shown in Fig. 1. (Results) In A-431 cells treated with compound A (-O-), the expression of HSP70 increased in a time-dependent manner during the period of from 6 hr to 12 hr after the treatment, and markedly decreased 24 hr after the treatment. The decrease was assumed to have been caused by the cytotoxic effect of compound A. On the other hand, in A-431 cells treated with gefinitib (-●-), the expression of HSP70 did not show a clear increase until after 12 hr, and the expression of HSP70 decreased after 24 hr from the treatment.

From the results, it has been clarified that compound A induces HSP70. Moreover, it has been clarified that HSP70 is induced after 6 hr from the treatment with compound A.

**Example 2** Induction of HSP70 in various human tumor-derived cells

(Method) As to the induction of HSP70 by compound A, induction in various human tumor cells was examined.

TE-8 cell, NCI-H520 cell, HPAC cell, DU145 cell, KPL-4 cell, MKN-45 cell, HL-60 cell and DLD-1 cell were each were cultured in a 12-well plate.

At the time point when the cells grew to about 70-80%, compound A and a control drug gefinitib were each added to 10 μM. DMSO (0.1%) was added to a control well. After the addition, the cells were collected 9 hr later and cell extracts were prepared. Expression of HSP70 protein in the tumor cell extracts was examined by Western blot using an HSP70 specific antibody.

The results are shown in Fig. 2.

(Results) HSP70 expression at 9 hr after compound A treatment increased in any human tumor-derived cells as compared to the DMSO-treated control. In contrast, the gefinitib-treated tumor cells did not show a clear increase in the HSP70 expression.

From the results, it has been clarified that compound A induces HSP70 in a broad range of tumor cells.

**Example 3** Suppressive action on HSP90 client protein expression

(Method) Effect of compound A on the expression of client proteins EGFR, HER2, ERα, AR, AKT and CDK4 of HSP90 chaperone was examined.

MCF-7 cells were cultured in a culture dish (diameter 10 cm) and, at the time point when the cells grew to about 70-80%, compound A and a control drug gefinitib were each added to 10 μM. Geldanamycin (1 μM) was added to the positive control and DMSO (0.1%) was added to the negative control.

After the addition of the compound, the cells were collected and divided in two 24 hr later. Cell extracts were prepared from half the cells, and cytoplasmic fraction and nuclear fraction were prepared from the remaining half. The cytoplasmic fraction and nuclear fraction were prepared using NER-PER (trademark) Nuclear and Cytoplasmic Extraction Reagents (manufactured by PIERCE, #78833). Expression of client in each tumor cell extract and nuclear fraction was examined by Western blot using an antibody specific to each client.

The results are shown in Fig. 3.

(Results) All clients showed decreased expression in the geldanamycin treatment group and compound A-treated cells, as compared to DMSO-treated control cells. In contrast, a clear decrease of client was not observed in the gefinitib-treated tumor cells. From the results, it has been suggested that compound A inhibits molecular chaperone function.

**Example 4** Suppressive effect on estradiol (E2)-dependent cell growth

(Method) Using human breast cancer-derived MCF-7 cell that expresses estrogen receptor and grows in response to estradiol (E2, 17β-Estradiol, Cat. #E 1024, Sigma Ltd.), the effect of compound A on the cell growth in the presence of E2 was examined. As the control compound, tamoxifen (Cancer Res., 62, 2474-2477, 2002) (Cat. # T 9262, Sigma Ltd.) known to suppress growth of human breast cancer-derived MCF-7 cell by E2 stimulation was used. MCF-7 cells were suspended in a phenol red non-addition DME-F12 mixed medium containing 5% fetal calf serum treated with activated carbon, and cultured at $2\times10^4$ cells/well in a 24 well plate. The next day (Day 1), E2 was added to half the number of wells in the plate to the final concentration of 10 nM and the medium alone was added to the rest of the wells during medium exchage. Compound A was added to the wells at 0.037, 0.11, 0.33, 1 and 3 μM, and tamoxifen was added to the wells at 0.062, 0.19, 0.56, 1.7 and 5 μM.

On Day 4 and Day 7, the medium was discarded, a medium having the same composition was added and the cells were continuously cultured. The medium alone was added to the control wells.

The cell growth activity was measured on Day 10 by the dye method using XTT.

That is, after the completion of the culture, XTT was added to each well and, after color development for 4 hr, the reaction mixture was transferred to a 96 well plate, and the absorbance at 492 nm was measured.

The suppression rate was calculated from the following formula.

Suppression rate (%) = {1- [(absorbance of compound addition well in the presence of E2)-(absorbance of control well without E2 addition)]÷[(absorbance of control well in the presence of E2)-(absorbance of control well without E2 addition)] ×100

The results are shown in Fig. 4. (Results) It has been clarified that compound A (-O-) suppresses the growth of human breast cancer-derived MCF-7 cells by E2 stimulation, as in the case of tamoxifen (-Δ-).

**Example 5** Cell growth suppression enhancing action by combined use of hyperthermic treatment and compound A

(Method) Using human prostate cancer-derived DU 145 cells, the effect of compound A on the cell growth suppressive action during hyperthermic treatment was examined. DU 145 cells were cultured at $1\times10^4$ cells/well in two 96 well plates. The next day, compound A was added to each plate to 0.37, 1.1, 3.3 and 10 μM, one plate was incubated for 4 hr in a 5% $CO_2$ incubator set to 43°C (heat treatment), and the other plate was incubated in the same manner for 4 hr in a 5% $CO_2$ incubator set to 37°C. After 4 hr, the wells were washed 3 times with saline, a growth medium was added and cultured in a 5% $CO_2$ incubator at 37°C for 24 hr.

The cell growth activity was measured by the dye method using XTT as in Example 4. That is, after the culture, XTT was added to each well and, 4 hr later, the absorbance at 492 nm was measured.

T/C (%) was determined from the following formula with the absorbance of the control well in the plate without the heat treatment as 100.

T/C (%) = [absorbance of heat-treated control well or compound-treated well ÷ absorbance of control well without heat treatment] ×100

The results are shown in Fig. 5.
(Results) From the results, it has been clarified that the cell death due to heat shock is enhanced by increasing the concentration of compound A.

**Example 6** Antitumor effect of compound A on human breast cancer-derived MCF-7 implanted in nude mouse

(Method) Human breast cancer-derived MCF-7 cells were subcutaneously implanted in famale nude mouse purchased from CLEA Japan, Inc. The effect of compound A on the growth was examined. A 17β-estradiol solution was added dropwise to a small piece of glass microfibre filter GF/B (Whatman, #1821 05) to give a pellet (500 μg/filter). The pellet was subcutaneously implanted in the neck of famale Balb/c nude mouse, and MCF-7 cells were subcutaneously implanted in the right back at $5\times10^6$ cells/mouse. From 7 days after the implantation, compound A was orally administered at 30 mg/kg and 100 mg/kg to nude mice (5 per group) once a day for 2 weeks.

The 0.5% tragacanth solution used as a solvent was similarly administered to the control. The next day of the final administration, the nude mice were sacrificed under anesthesia, and the subcutaneous tumor was isolated and weighed. The mean tumor weight and standard error of each group were calculated, and statistically analyzed by the Dunnett's test and using the solvent administration group as a control.

The results are shown in Fig. 6.
(Results) As shown in Fig. 6, the tumor weight significantly decreased in the group administered with 100 mg/kg of compound A as compared to a control group.

From the results, it has been clarified that compound A shows an antitumor effect on MCF-7 breast cancer grown with estrogen.

**Example 7** Induction of HSP70 by compounds A - F using human breast cancer-derived T-47D cells

(Method) T-47D cells were cultured in a 12-well plate and, at the time point when the cells grew to about 70-80%, compounds A, B, C, D, E and F were each added to 10 μM.

DMSO (0.1%) was added to the control. After the addition, the cells were collected 9 hr later and cell extracts were prepared. Expression of HSP70 protein in the cell extracts was examined by Western blot using an HSP70 specific antibody. T/C (%) was determined using the amount of chemical luminescence on a PVDF film due to an HRP-labeled secondary antibody as the HSP70 expression amount and HSP70 expression amount of the cells in the control well as 100.

The results are shown in Table 10.

[Table 10]

| compound | Experiment 1 (T/C, %) | Experiment 2 (T/C, %) |
|---|---|---|
| control | 100 | 100 |
| A | 299 | 895 |
| B | | 482 |
| C | 207 | |
| D | | 446 |
| E | 172 | |
| F | 141 | |

(Results) Expression of HSP70 increased in T-47D cells treated with compounds A - F, as compared to the control.

From the results, it has been shown that HSP70 is induced in the cell by treating T-47D cells with compounds A - F for 9 hr.

**Example 8** Induction of ubiquitinated protein by compound A in human breast cancer-derived T-47D cell

(Method) T-47D cells were cultured in a 6-well plate and, at the time point when the cells grew to about 70-80%, compound A was added to 1, 3 and 10 μM and bortezomib (PS-341) and geldanamycin (GA) were each added to 3 μM. DMSO (0.1%) was added to the control. After the addition, the cells were collected 1, 3, 6 and 24 hr later and cell extracts were prepared. Expression of ubiquitinated protein in the cell extracts was examined by Western blot using a ubiquitin specific antibody. T/C (%) was determined using the amount of chemical luminescence on a PVDF film due to an HRP-labeled secondary antibody as the ubiquitinated protein expression amount and the expression amount of the cell extract in the control well as 100.

The results are shown in Fig. 7.

(Results) In bortezomib (-O-)-treated T-47D cells, a ubiquitinated protein remarkably appeared from 3 hr after the treatment. In T-47D cells treated with compound A 1 μM (-●-) and geldanamycin (-□-), ubiquitination of protein did not occur until after 24 hr. In contrast, in T-47D cells treated with compound A 3 (-▲-) μM and 10 μM (-■-), the amount of ubiquitinated protein increased from 6 hr after the treatment, which lasted until after 24 hr from the treatment.

From the results, it has been clarified that ubiquitinated protein appears from 6 hr later, by treating with compound A (3 μM and 10 μ).

Industrial Applicability

According to the present invention, a molecular chaperone function regulator comprising a quinazoline derivative represented by the aforementioned formula (I) or a pharmaceutically acceptable salt thereof, a hydrate or solvate thereof, an optically active form or racemate thereof or a diastereomer mixture thereof as an active ingredient can be provided.

This application is filed claiming priority right based on a patent application No. 2004-254716 filed in Japan.

## Claims

1. A molecular chaperone function regulator comprising, as an active ingredient, a quinazoline derivative represented by the following formula (I)

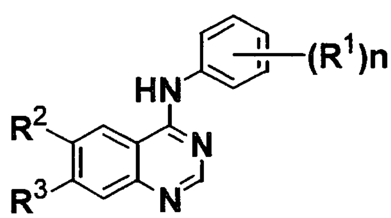

( I )

wherein n is an integer of 0 to 3, $R^1$ is a hydrogen atom, a halogen atom, hydroxy, cyano, nitro, trifluoromethyl, $C_{1-5}$

alkyl, $C_{1-5}$ alkoxy, $-S(O)_fR^{13}$ (wherein f is an integer of 0 to 2, and $R^{13}$ is $C_{1-5}$ alkyl), $-NR^{14}R^{15}$ (wherein $R^{14}$ and $R^{15}$ are each independently a hydrogen atom, $C_{1-5}$ alkyl, $C_{1-5}$ alkanoyl or $C_{1-5}$ alkylsulfonyl), $C_{2-5}$ alkenyl, $C_{2-5}$ alkynyl or $C_{1-5}$ alkanoyl, one of $R^2$ and $R^3$ is $R^{27}SO_2NH$- (wherein $R^{27}$ is $C_{1-5}$ alkyl optionally substituted by morpholino), $(R^{28}SO_2)_2N$- (wherein $R^{28}$ is $C_{1-5}$ alkyl optionally substituted by morpholino), $C_{1-5}$ alkoxy, $CH_3COCH_2CONH$-, $CH_3SCH_2CH_2CONH$-, $NCCH_2CONH$-,

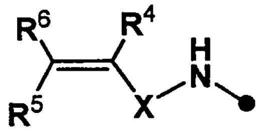

or

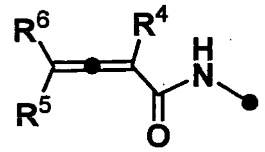

(wherein X is -C(O)- or $SO_2$- and $R^4$, $R^5$ and $R^6$ are each independently a hydrogen atom, a halogen atom or $C_{1-5}$ alkyl optionally substituted by a halogen atom, morpholino, 4-$C_{1-5}$ alkylpiperazin-1-yl or di($C_{1-5}$ alkyl)amino), or

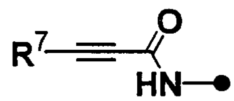

(wherein $R^7$ is $C_{1-5}$ alkyl optionally substituted by a hydrogen atom, a halogen atom, morpholino, 4-$C_{1-5}$ alkylpiperazin-1-yl or di($C_{1-5}$ alkyl)amino), and
the other of $R^2$ and $R^3$ is

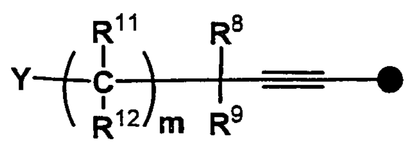

or

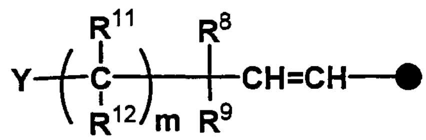

wherein $R^8$ and $R^9$ are each independently [a] a hydrogen atom, [b] $C_{1-5}$ alkyl optionally substituted by hydroxy or $C_{1-5}$ alkoxy,
[c] $R^8$ and $R^9$ in combination show C=O, or [d] $R^8$ and $R^9$ in combination form a ring to show $C_{3-8}$ cycloalkylene optionally via -O-, -S- or $-NR^{10}$- (wherein $R^{10}$ is a hydrogen atom or $C_{1-5}$ alkyl), m is an integer of 0 to 3, $R^{11}$ and $R^{12}$ are each independently a hydrogen atom or $C_{1-5}$ alkyl, and
Y is a hydrogen atom, hydroxy, $C_{1-5}$ alkoxy, $C_{1-5}$ alkanoyloxy, $-N(R^{16})-(CO)u-(CR^{17}R^{18})v-(CO)j-R^{19}$ (wherein $R^{16}$ is [a] a hydrogen atom or [b] $C_{1-5}$ alkyl optionally substituted by cyano or $C_{1-5}$ alkoxy, $R^{17}$ and $R^{18}$ are each independently a hydrogen atom or $C_{1-5}$ alkyl, u and j are each 0 or 1, v is an integer of 1 to 5, and $R^{19}$ is a hydrogen atom, hydroxy, cyano, amino, $C_{1-5}$ alkoxy, morpholino, 4-$C_{1-5}$ alkylpiperazin-1-yl or di($C_{1-5}$ alkyl) amino,
provided that when (i) u and j are simultaneously 0, then v is an integer of 2 to 5, and when (ii) $R^{19}$ is a cyano group, then j is 0),

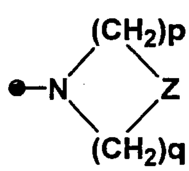

wherein p and q are each independently 2 or 3, and Z is -O-, $-S(O)_g$- (wherein g is an integer of 0 to 2), carbonyl or $-NR^{20}$-(wherein $R^{20}$ is [a] a hydrogen atom, [b] $C_{1-5}$ alkylsulfonyl, [c] $C_{1-5}$ alkanoyl, [d] $C_{1-5}$ alkoxycarbonyl, or [e] $C_{1-5}$ alkyl optionally substituted by cyano or $C_{1-5}$ alkoxy), or

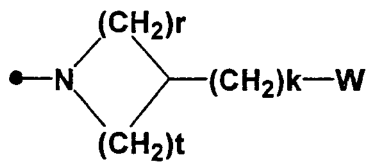

wherein r and t are each independently an integer of 1 to 3, k is 0 or 1, and W is a hydrogen atom, hydroxy, $C_{1-5}$ alkoxy, $C_{1-5}$ alkanoyloxy, carboxyl, cyano, di($C_{1-5}$ alkyl)amino, morpholino, pyrrolidin-1-yl, piperidin-1-yl, 4-$C_{1-5}$ alkylpiperazin-1-yl or $CONR^{21}R^{22}$ (wherein $R^{21}$ and $R^{22}$ are each independently a hydrogen atom or $C_{1-5}$ alkyl), or a pharmaceutically acceptable salt thereof, or a hydrate or solvate thereof, or an optically active form or racemate thereof or a diastereomer mixture thereof.

**2.** The molecular chaperone function regulator of claim 1, wherein, in the quinazoline derivative of the aforementioned formula (I),
$R^2$ is $R^{27}SO_2NH$- (wherein $R^{27}$ is $C_{1-5}$ alkyl optionally substituted by morpholino), $(R^{28}SO_2)_2N$- (wherein $R^{28}$ is $C_{1-5}$ alkyl optionally substituted by morpholino), $C_{1-5}$ alkoxy, $CH_3COCH_2CONH$-, $CH_3SCH_2CH_2CONH$-, $N{\equiv}CCH_2CONH$-, or

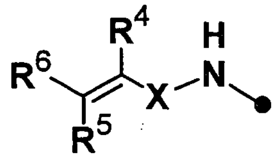

(wherein X is -C(O)- or $SO_2$-, and $R^4$, $R^5$and $R^6$ are each independently a hydrogen atom, a halogen atom or $C_{1-5}$ alkyl optionally substituted by a halogen atom, morpholino, 4-$C_{1-5}$ alkylpiperazin-1-yl or di($C_{1-5}$ alkyl)amino), and $R^3$ is

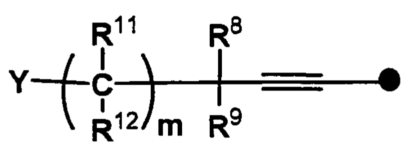

or

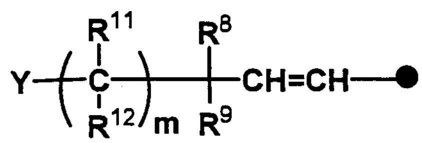

wherein m is an integer of 0 to 3,
$R^8$ and $R^9$ are each independently a hydrogen atom, hydroxy or $C_1$-5 alkyl optionally substituted by $C_{1-5}$ alkoxy, $R^8$ and $R^9$ in combination show -C(O)-, or $R^8$ and $R^9$ in combination form a ring to show $C_{3-8}$ cycloalkylene optionally via -O-, -S- or - $NR^{10}$- (wherein $R^{10}$ is a hydrogen atom or $C_{1-5}$ alkyl),
$R^{11}$ and $R^{12}$ are each independently a hydrogen atom or $C_{1-5}$ alkyl, and
Y is a hydrogen atom, hydroxy, $C_{1-5}$ alkoxy, $C_{1-5}$ alkanoyloxy, - $N(R^{16})$-(CO)u-$(CR^{17}R^{18})$v-(CO)j-$R^{19}$ (wherein $R^{16}$ is a hydrogen atom, or $C_{1-5}$ alkyl optionally substituted by cyano or $C_{1-5}$ alkoxy,
$R^{17}$ and $R^{18}$ are each independently a hydrogen atom or $C_{1-5}$ alkyl, u and j are each independently 0 or 1, v is an integer of 1 to 5, and
$R^{19}$ is a hydrogen atom, hydroxy, cyano, amino, $C_{1-5}$ alkoxy, morpholino, 4-$C_{1-5}$ alkylpiperazin-1-yl, or di($C_{1-5}$ alkyl) amino,
provided that when (i) u and j are simultaneously 0, then v is an integer of 2 to 5, and when (ii) $R^{19}$ is cyano, then j is 0),

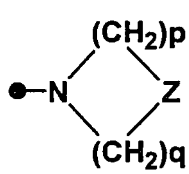

wherein p and q are each independently 2 or 3, and Z is -O-, - $S(O)_g$- (wherein g is an integer of 0 to 2), -C(O)- or

$-NR^{20}$-(wherein $R^{20}$ is a hydrogen atom, $C_{1-5}$ alkylsulfonyl, $C_{1-5}$ alkanoyl, $C_{1-5}$ alkoxycarbonyl, or $C_{1-5}$ alkyl optionally substituted by cyano or $C_{1-5}$ alkoxy), or

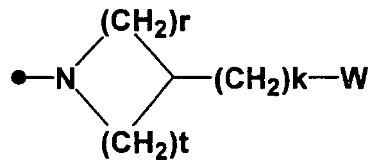

wherein r and t are each independently an integer of 1 to 3, k is 0 or 1, and W is a hydrogen atom, hydroxy, $C_{1-5}$ alkoxy, $C_{1-5}$ alkanoyloxy, carboxyl, cyano, di($C_{1-5}$ alkyl)amino, morpholino, pyrrolidin-1-yl, piperidin-1-yl, 4-$C_{1-5}$ alkylpiperazin-1-yl or $CONR^{21}R^{22}$ (wherein $R^{21}$ and $R^{22}$ are each independently a hydrogen atom or $C_{1-5}$ alkyl) .

**3.** The molecular chaperone function regulator of claim 1 or 2, wherein, in the quinazoline derivative of the aforementioned formula (I),
$R^3$ is

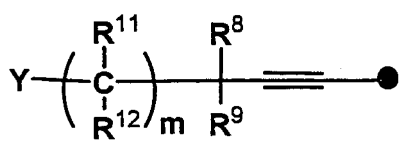

or

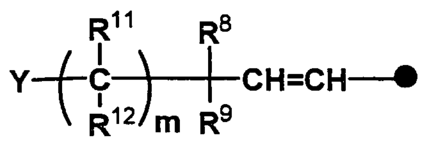

wherein m is an integer of 0 to 3,
$R^8$ and $R^9$ are each independently a hydrogen atom or $C_{1-5}$ alkyl optionally substituted by $C_{1-5}$ alkoxy, or $R^8$ and $R^9$ in combination form a ring to show $C_{3-8}$ cycloalkylene optionally via -O- or -NH-,
$R^{11}$ and $R^{12}$ are each independently a hydrogen atom or $C_{1-5}$ alkyl, and
Y is a hydrogen atom, hydroxy, $C_{1-5}$ alkoxy, $C_{1-5}$ alkanoyloxy, - $N(R^{16})$-(CO)u-$(CR^{17}R^{18})$v-(CO)j-$R^{19}$ (wherein $R^{16}$ is a hydrogen atom, or $C_{1-5}$ alkyl optionally substituted by cyano or $C_{1-5}$ alkoxy,
$R^{17}$ and $R^{18}$ are each independently a hydrogen atom or $C_{1-5}$ alkyl, u and j are each independently 0 or 1, v is an integer of 1 to 5, and $R^{19}$ is a hydrogen atom, hydroxy, cyano, amino, $C_{1-5}$ alkoxy, morpholino, 4-$C_{1-5}$ alkylpiperazin-1-yl, or di($C_{1-5}$ alkyl)amino, provided that when (i) u and j are simultaneously 0, then v is an integer of 2 to 5, and when (ii) $R^{19}$ is cyano, then j is 0),

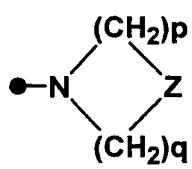

wherein p and q are each independently 2 or 3, and Z is -O-, - C(O)- or $-NR^{20}$- (wherein $R^{20}$ is a hydrogen atom, $C_{1-5}$ alkylsulfonyl, $C_{1-5}$ alkanoyl, $C_{1-5}$ alkoxycarbonyl, or $C_{1-5}$ alkyl optionally substituted by cyano or $C_{1-5}$ alkoxy), or

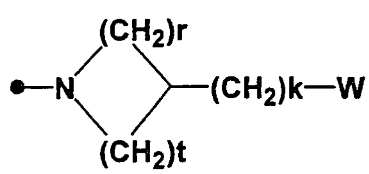

wherein r and t are each independently an integer of 1 to 3, k is 0 or 1, and W is a hydrogen atom, hydroxy, $C_{1-5}$ alkoxy, $C_{1-5}$ alkanoyloxy, carboxyl, cyano, di($C_{1-5}$ alkyl)amino, morpholino or $CONR^{21}R^{22}$ (wherein $R^{21}$ and $R^{22}$ are each independently a hydrogen atom or $C_{1-5}$ alkyl) .

4. The molecular chaperone function regulator of any one of claims 1 to 3, wherein, in the quinazoline derivative of the aforementioned formula (I),
$R^3$ is

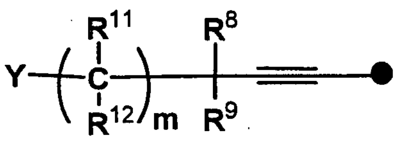

or

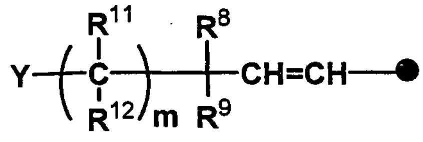

wherein m is an integer of 0 or 1,
$R^8$ and $R^9$ are each independently a hydrogen atom or $C_{1-5}$ alkyl optionally substituted by $C_{1-5}$ alkoxy, or $R^8$ and $R^9$ in combination form a ring to show $C_{3-8}$ cycloalkylene optionally via -O- or -NH-, and
Y is $C_{1-5}$ alkoxy, $-N(R^{16})-(CO)u-(CH_2)_2-(CO)j-R^{19}$ (wherein u and j are each 0 or 1, $R^{16}$ is a hydrogen atom, or $C_{1-5}$ alkyl optionally substituted by $C_{1-5}$ alkoxy, and $R^{19}$ is a hydrogen atom, cyano, $C_{1-5}$ alkoxy, morpholino, 4-$C_{1-5}$ alkylpiperazin-1-yl or di($C_{1-5}$ alkyl) amino, provided that when $R^{19}$ is cyano, then j is 0),

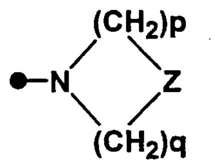

wherein p and q are each independently 2 or 3, and Z is -O- or $-NR^{20}-$ (wherein $R^{20}$ is a hydrogen atom, $C_{1-5}$ alkylsulfonyl, $C_{1-5}$ alkanoyl, $C_{1-5}$ alkoxycarbonyl, or $C_{1-5}$ alkyl optionally substituted by cyano or $C_{1-5}$ alkoxy), or

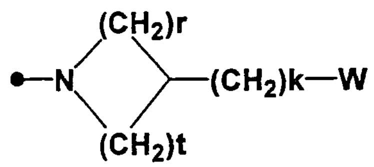

wherein r and t are each independently 1 or 2, and
W is a hydrogen atom, hydroxy, $C_{1-5}$ alkoxy, $C_{1-5}$ alkanoyloxy or carboxyl.

5. The molecular chaperone function regulator of any one of claims 1 to 4, wherein, in the quinazoline derivative of the aforementioned formula (I),
$R^3$ is

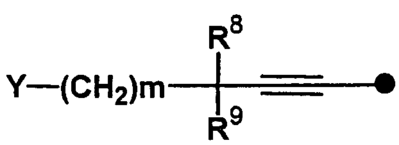

wherein m is 0 or 1,
$R^8$ and $R^9$ are each independently a hydrogen atom or $C_{1-5}$ alkyl optionally substituted by $C_{1-5}$ alkoxy, or $R^8$ and $R^9$ in combination form a ring to show $C_{3-8}$ cycloalkylene optionally
via -O- or -NH-, and
Y is $C_{1-5}$ alkoxy, $-N(R^{16})-(CH_2)_2-R^{19}$ (wherein $R^{16}$ is a hydrogen atom or $C_{1-5}$ alkyl optionally substituted by $C_{1-5}$ alkoxy, and $R^{19}$
is a hydrogen atom, cyano, $C_{1-5}$ alkoxy, morpholino, 4-$C_{1-5}$ alkylpiperazin-1-yl, or di($C_{1-5}$ alkyl)amino, provided that when $R^{19}$ is cyano, then j is 0),

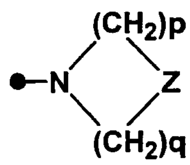

wherein p and q are each independently 2 or 3, and
Z is -O- or -$NR^{20}$- (wherein $R^{20}$ is a hydrogen atom, $C_{1-5}$ alkylsulfonyl, $C_{1-5}$ alkanoyl, $C_{1-5}$ alkoxycarbonyl, or $C_{1-5}$ alkyl optionally substituted by cyano or $C_{1-5}$ alkoxy), or

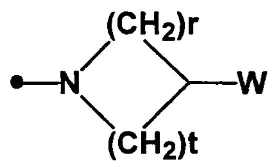

wherein r and t are each independently 1 or 2, and
W is a hydrogen atom, hydroxy, $C_{1-5}$ alkoxy, $C_{1-5}$ alkanoyloxy, carboxyl.

6. The molecular chaperone function regulator of any one of claims 1 to 5, wherein, in the quinazoline derivative of the aforementioned formula (I),
$R^2$ is $R^{27}SO_2NH$- (wherein $R^{27}$ is $C_{1-5}$ alkyl), $C_{1-5}$ alkoxy or

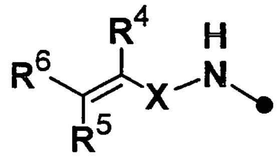

wherein each symbol is as defined above.

7. The molecular chaperone function regulator of any one of claims 1 to 6, wherein, in the quinazoline derivative of the aforementioned formula (I), n is 1 or 2, and $R^1$ is a halogen atom, cyano, $C_{1-5}$ alkyl, $C_{1-5}$ alkoxy, -$NR^{14}R^{15}$ (wherein $R^{14}$ and $R^{15}$ are each independently a hydrogen atom, $C_{1-5}$ alkyl, $C_{1-5}$ alkanoyl or $C_{1-5}$ alkylsulfonyl), $C_{2-5}$ alkynyl, or $C_{1-5}$ alkanoyl.

8. The molecular chaperone function regulator of any one of claims 1 to 7, wherein, in the quinazoline derivative of the aforementioned formula (I), n is 2, $R^1$ is a halogen atom, $R^2$ is

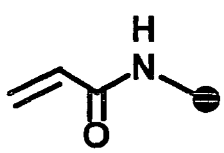

,

and
$R^3$ is

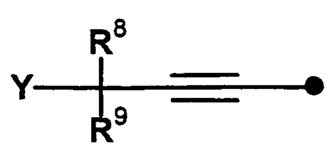

wherein $R^8$ and $R^9$ are each $C_{1-5}$ alkyl optionally substituted by $C_{1-5}$ alkoxy, and Y is $-N(R^{16})-(CH_2)_2-R^{19}$ (wherein $R^{16}$ is $C_{1-5}$ alkyl optionally substituted by $C_{1-5}$ alkoxy, and $R^{19}$ is di ($C_{1-5}$ alkyl) amino), or

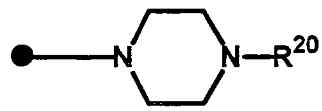

wherein $R^{20}$ is $C_{1-5}$ alkyl optionally substituted by $C_{1-5}$ alkoxy.

**9.** The molecular chaperone function regulator of any one of claims 1 to 8, wherein the quinazoline derivative is N-{4-[(3-chloro-4-fluorophenyl)amino]-7-[3-methyl-3-(4-methyl-1-piperazinyl)-1-butynyl]-6-quinazolinyl}acrylamide bis(4-methylbenzenesulfonate).

**10.** A pharmaceutical composition for the prophylaxis and/or treatment of a disease involving a heat shock protein, or for a thermotherapy enhancing action, which comprises the molecular chaperone function regulator of any one of claims 1 to 9.

**11.** The pharmaceutical composition of claim 10, wherein the disease involving a heat shock protein is a hormone-dependent disease, a hematopoietic tumor or a malignant soft tissue tumor.

**12.** The pharmaceutical composition of claim 11, wherein the hormone-dependent disease is prostatic hyperplasia, endometriosis or a hormone-sensitive cancer.

**13.** The pharmaceutical composition of claim 12, wherein the hormone-sensitive cancer is a hormone-sensitive breast cancer, a hormone-sensitive prostate cancer or a hormone-sensitive endometrial cancer.

**14.** The pharmaceutical composition of claim 11, wherein the hematopoietic tumor is leukemia, malignant lymphoma or myeloma.

**15.** The pharmaceutical composition of claim 11, wherein the malignant soft tissue tumor is osteosarcoma or soft tissue sarcoma.

**16.** A pharmaceutical composition for the prophylaxis and/or treatment of a disease involving a heat shock protein, or for a thermotherapy enhancing action, which comprises a quinazoline derivative represented by the following formula (I)

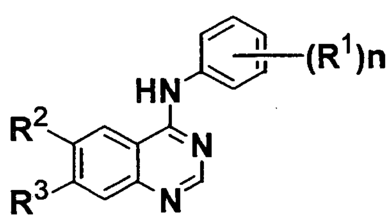

( I )

wherein n is an integer of 0 to 3, $R^1$ is a hydrogen atom, a halogen atom, hydroxy, cyano, nitro, trifluoromethyl, $C_{1-5}$ alkyl, $C_{1-5}$ alkoxy, $-S(O)_fR^{13}$ (wherein f is an integer of 0 to 2, and $R^{13}$ is $C_{1-5}$ alkyl), $-NR^{14}R^{15}$ (wherein $R^{14}$ and $R^{15}$ are each independently a hydrogen atom, $C_{1-5}$ alkyl, $C_{1-5}$ alkanoyl or $C_{1-5}$ alkylsulfonyl), $C_{2-5}$ alkenyl, $C_{2-5}$ alkynyl or $C_{1-5}$ alkanoyl, one of $R^2$ and $R^3$ is $R^{27}SO_2NH-$ (wherein $R^{27}$ is $C_{1-5}$ alkyl optionally substituted by morpholino), $(R^{28}SO_2)_2N-$ (wherein $R^{28}$ is $C_{1-5}$ alkyl optionally substituted by morpholino), $C_{1-5}$ alkoxy, $CH_3COCH_2CONH-$, $CH_3SCH_2CH_2CONH-$, $NCCH_2CONH-$,

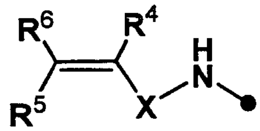

or

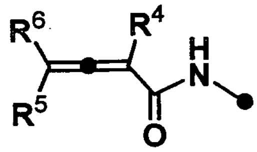

(wherein X is -C(O)- or $SO_2$-, and $R^4$, $R^5$ and $R^6$ are each independently a hydrogen atom, a halogen atom or $C_{1-5}$ alkyl optionally substituted by a halogen atom, morpholino, 4-$C_{1-5}$ alkylpiperazin-1-yl or di ($C_{1-5}$ alkyl)amino), or

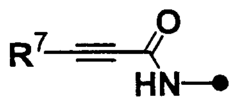

(wherein $R^7$ is $C_{1-5}$ alkyl optionally substituted by a hydrogen atom, a halogen atom, morpholino, 4-$C_{1-5}$ alkylpiperazin-1-yl or di($C_{1-5}$ alkyl)amino), and
the other of $R^2$ and $R^3$ is

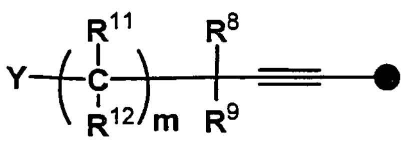

or

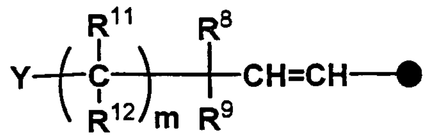

wherein $R^8$ and $R^9$ are each independently [a] a hydrogen atom, [b] $C_{1-5}$ alkyl optionally substituted by hydroxy or $C_{1-5}$ alkoxy, [c] $R^8$ and $R^9$ in combination show C=O, or [d] $R^8$ and $R^9$ in combination form a ring to show $C_{3-8}$ cycloalkylene optionally via -O-, -S- or -$NR^{10}$- (wherein $R^{10}$ is a hydrogen atom or $C_{1-5}$ alkyl), m is an integer of 0 to 3, $R^{11}$ and $R^{12}$ are each independently a hydrogen atom or $C_{1-5}$ alkyl, and
Y is a hydrogen atom, hydroxy, $C_{1-5}$ alkoxy, $C_{1-5}$ alkanoyloxy, - N($R^{16}$)-(CO)u-($CR^{17}R^{18}$)v-(CO)j-$R^{19}$ (wherein $R^{16}$ is [a] a hydrogen atom or [b] $C_{1-5}$ alkyl optionally substituted by cyano or $C_{1-5}$ alkoxy, $R^{17}$ and $R^{18}$ are each independently a hydrogen atom or $C_{1-5}$
alkyl, u and j are each 0 or 1, v is an integer of 1 to 5, and $R^{19}$ is a hydrogen atom, hydroxy, cyano, amino, $C_{1-5}$ alkoxy, morpholino, 4-$C_{1-5}$ alkylpiperazin-1-yl or di($C_{1-5}$ alkyl)amino,
provided that when (i) u and j are simultaneously 0, then v is an integer of 2 to 5, and when (ii) $R^{19}$ is a cyano group, then j is 0),

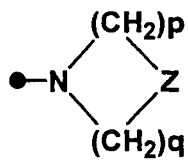

wherein p and q are each independently 2 or 3, and Z is -O-, - $S(O)_g$- (wherein g is an integer of 0 to 2), carbonyl or -$NR^{20}$-(wherein $R^{20}$ is [a] a hydrogen atom, [b] $C_{1-5}$ alkylsulfonyl, [c]
$C_{1-5}$ alkanoyl, [d] $C_{1-5}$ alkoxycarbonyl, or [e] $C_{1-5}$ alkyl optionally substituted by cyano or $C_{1-5}$ alkoxy), or

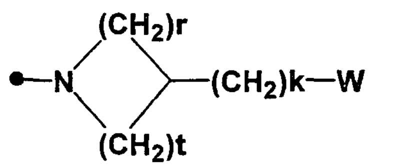

wherein r and t are each independently an integer of 1 to 3, k is 0 or 1, and W is a hydrogen atom, hydroxy, $C_{1-5}$

alkoxy, $C_{1-5}$ alkanoyloxy, carboxyl, cyano, di($C_{1-5}$ alkyl)amino, morpholino, pyrrolidin-1-yl, piperidin-1-yl, 4-$C_{1-5}$ alkylpiperazin-1-yl or $CONR^{21}R^{22}$ (wherein $R^{21}$ and $R^{22}$ are each independently a hydrogen atom or $C_{1-5}$ alkyl), or a pharmaceutically acceptable salt thereof, a hydrate or solvate thereof, an optically active form or racemate thereof or a diastereomer mixture thereof.

**17.** The pharmaceutical composition of claim 16, wherein the disease involving a heat shock protein is a hormone-dependent disease, a hematopoietic tumor, or a malignant soft tissue tumor.

**18.** The pharmaceutical composition of claim 17, wherein the hormone-dependent disease is prostatic hyperplasia, endometriosis or a hormone-sensitive cancer.

**19.** The pharmaceutical composition of claim 18, wherein the hormone-sensitive cancer is a hormone-sensitive breast cancer, a hormone-sensitive prostate cancer or a hormone-sensitive endometrial cancer.

**20.** The pharmaceutical composition of claim 17, wherein the hematopoietic tumor is leukemia, malignant lymphoma or myeloma.

**21.** The pharmaceutical composition of claim 17, wherein the malignant soft tissue tumor is osteosarcoma or soft tissue sarcoma.

FIG. 1

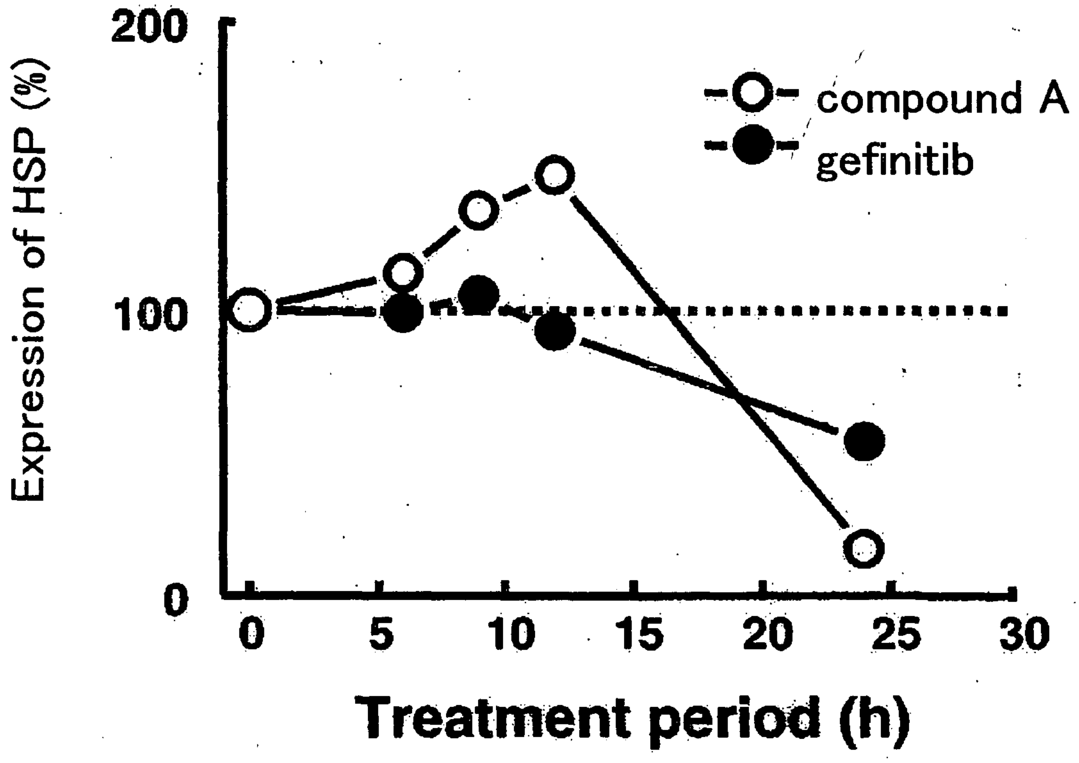
200
100
0
Expression of HSP (%)
compound A
gefinitib
0
5
10
15
20
25
30
Treatment period (h)

FIG. 2

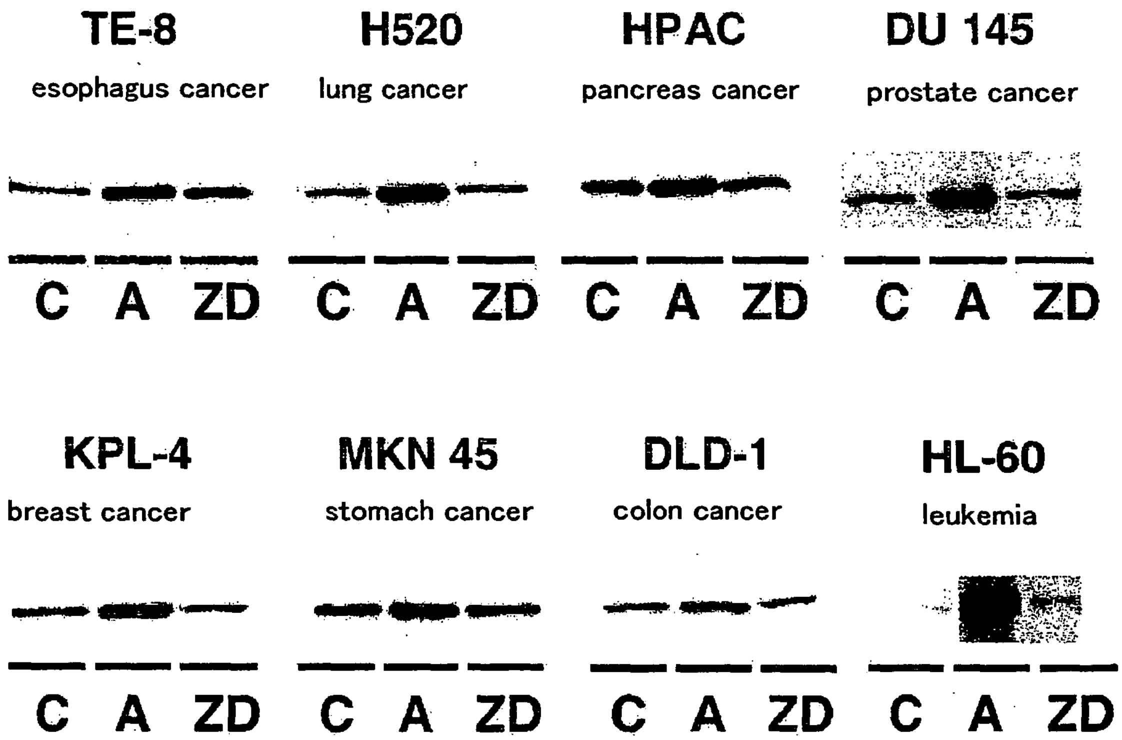
TE-8
esophagus cancer
C A ZD
H520
lung cancer
C A ZD
HPAC
pancreas cancer
C A ZD
DU 145
prostate cancer
C A ZD
KPL-4
breast cancer
C A ZD
MKN 45
stomach cancer
C A ZD
DLD-1
colon cancer
C A ZD
HL-60
leukemia
C A ZD

FIG. 3

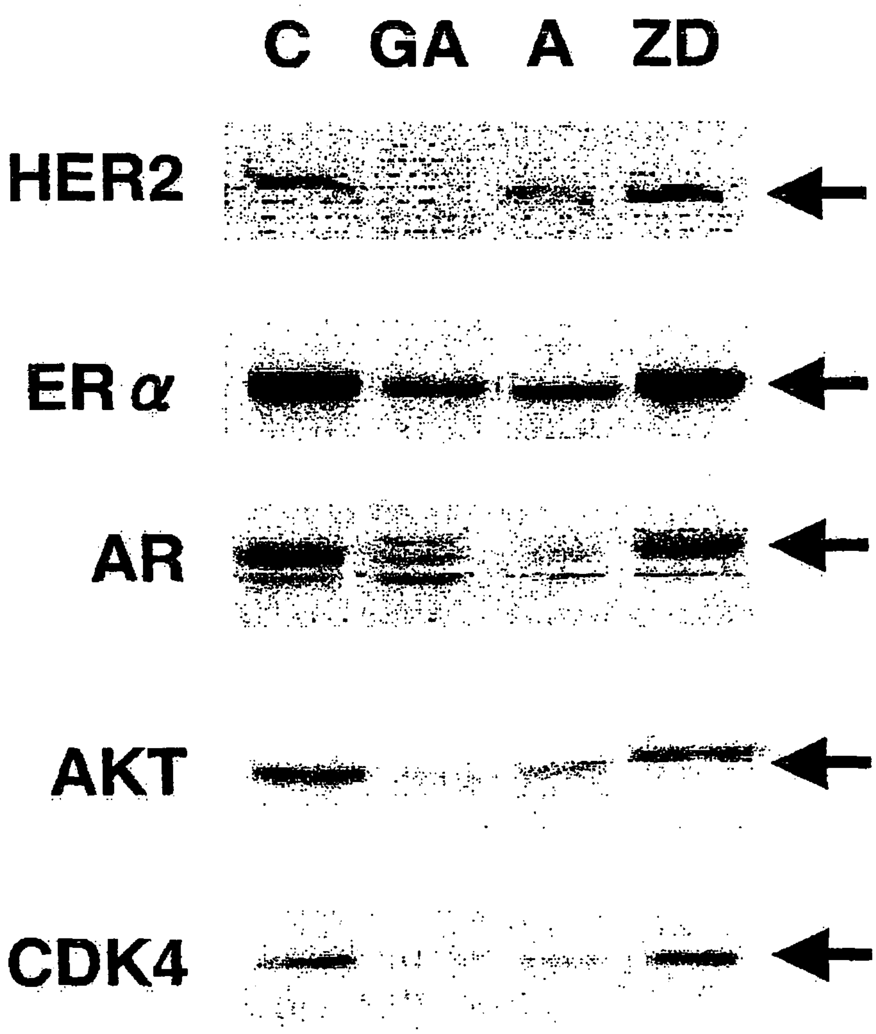

FIG. 4

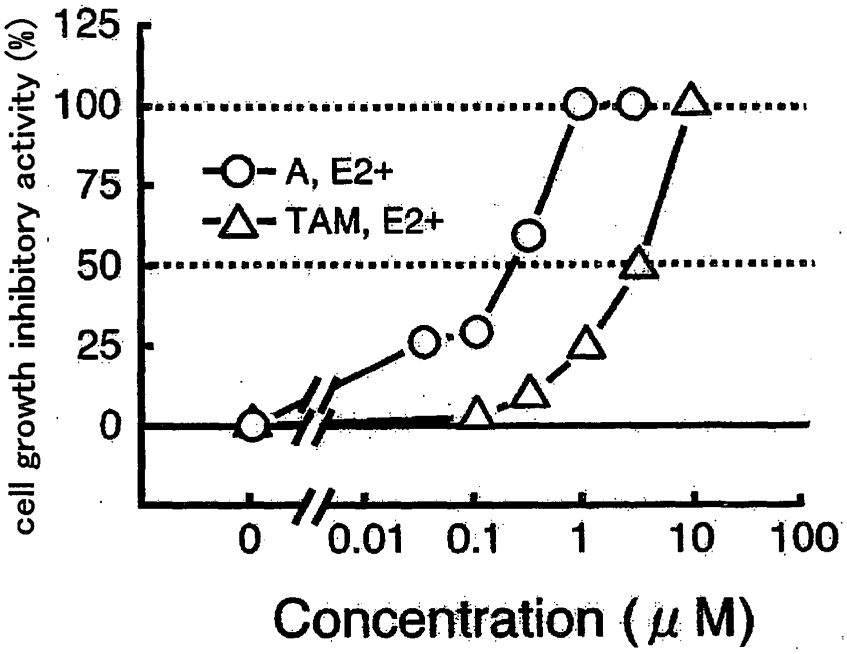
cell growth inhibitory activity (%)
125
100
75
50
25
0
-O- A, E2+
-Δ- TAM, E2+
0
0.01
0.1
1
10
100
Concentration (μM)

FIG. 5

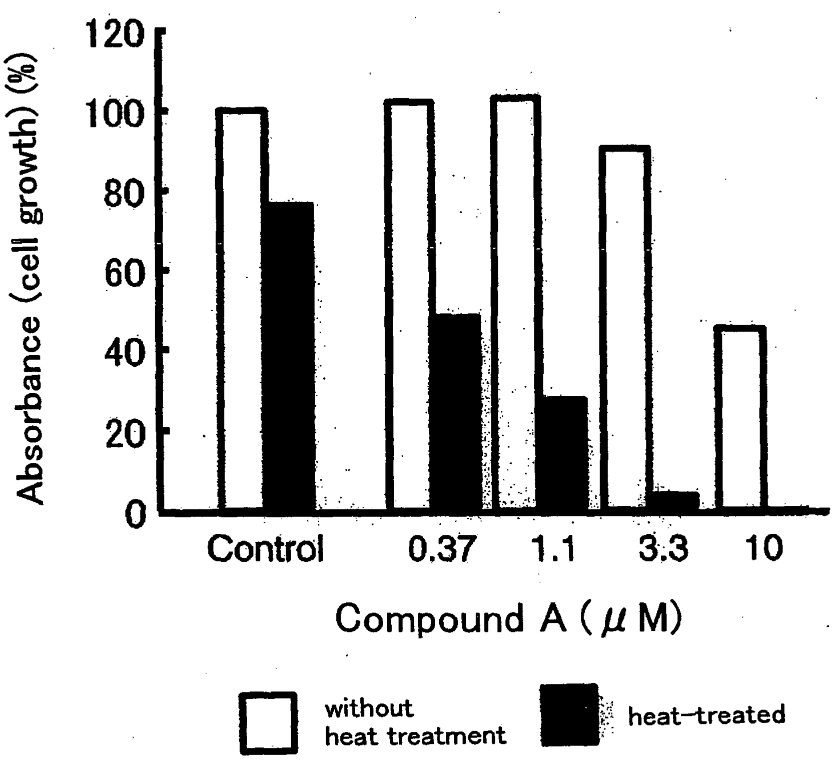
Absorbance (cell growth) (%)
120
100
80
60
40
20
0
Control
0.37
1.1
3.3
10
Compound A (μM)
without heat treatment
heat-treated

FIG. 6

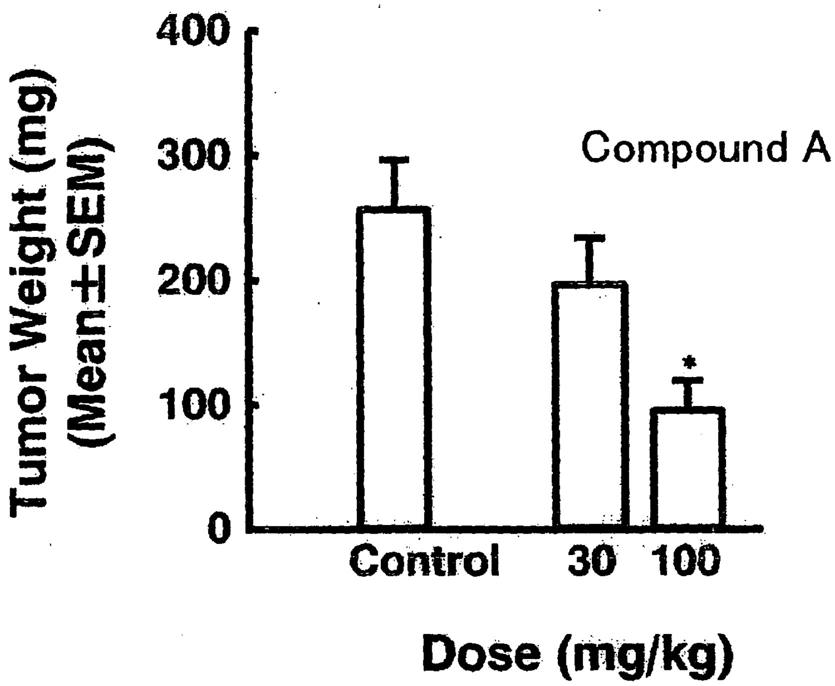
400
300
200
100
0
Tumor Weight (mg)
(Mean±SEM)
Compound A
*
Control
30
100
Dose (mg/kg)

FIG. 7

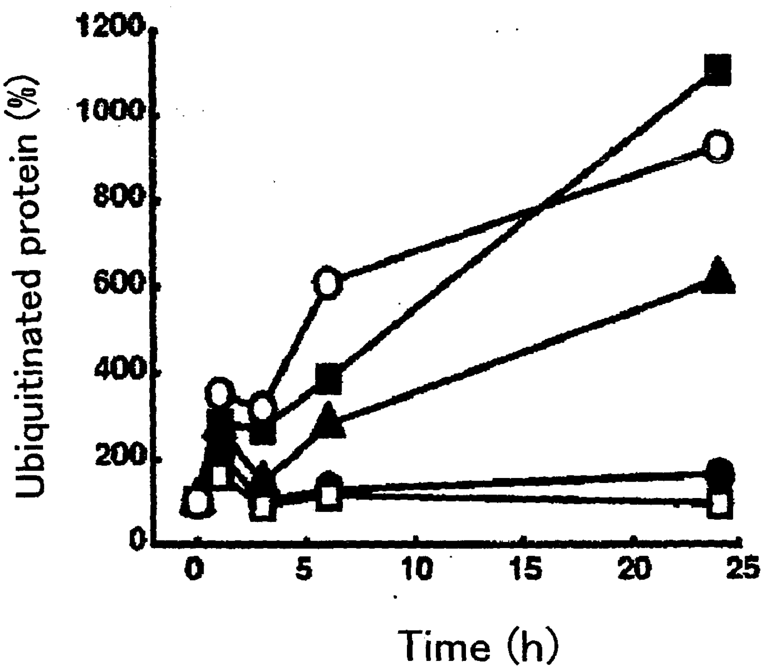
1200
1000
800
600
400
200
0
Ubiquitinated protein (%)
0
5
10
15
20
25
Time (h)

**INTERNATIONAL SEARCH REPORT**

International application No.
PCT/JP2005/016007

A. CLASSIFICATION OF SUBJECT MATTER
***C07D239/94***(2006.01), ***A61K31/517***(2006.01), ***A61P13/08***(2006.01), ***A61P15/00***(2006.01), ***A61P35/00***(2006.01), ***A61P35/02***(2006.01), ***A61P43/00***(2006.01)

According to International Patent Classification (IPC) or to both national classification and IPC

B. FIELDS SEARCHED

Minimum documentation searched (classification system followed by classification symbols)
***C07D239/94***(2006.01), ***A61K31/517***(2006.01), ***A61P13/08***(2006.01), ***A61P15/00***(2006.01), ***A61P35/00***(2006.01), ***A61P35/02***(2006.01), ***A61P43/00***(2006.01)

Documentation searched other than minimum documentation to the extent that such documents are included in the fields searched

Electronic data base consulted during the international search (name of data base and, where practicable, search terms used)
CAPLUS(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| X | WO 02/66445 A1 (Mitsubishi Pharma Corp.), 29 August, 2002 (29.08.02), Full text; particularly, page 1, line 21 to page 3, line 9; pages 14 to 22; tables 1 to 9; page 31, line 2 to page 33, line 9; pages 124 to 130; test examples; page 131 to 148; Claims & EP 1369418 A1 & KR 2003086269 A & AU 2002233682 A1 & US 2004116422 A1 & CN 1492860 A | 1-21 |
| A | Dunn, F. B. Heat Shock Protein Inhibitor Shows Antitumor Activity, Journal of the National Cancer Institute, 21 August, 2002 (21.08.02), Vol.94, No.16, pages 1194 to 1195 | 1-21 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 30 September, 2005 (30.09.05) | 18 October, 2005 (18.10.05) |
| Name and mailing address of the ISA/ Japanese Patent Office | Authorized officer |
| Facsimile No. | Telephone No. |

Form PCT/ISA/210 (second sheet) (January 2004)

**INTERNATIONAL SEARCH REPORT**

International application No.
PCT/JP2005/016007

C (Continuation). DOCUMENTS CONSIDERED TO BE RELEVANT

| Category* | Citation of document, with indication, where appropriate, of the relevant passages | Relevant to claim No. |
|---|---|---|
| A | WO 02/094196 A2 (SLOAN-KTTERING INSTITUTE FOR CANCER RESEARCH), 28 November, 2002 (28.11.02), Full text; particularly, pages 1 to 3<br>& EP 1404871 A2 & US 2004266746 A1<br>& JP 2004-529188 A | 1-21 |
| A | WO 03/037860 A2 (CONFORMA THERAPEUTICS CORP.), 08 May, 2003 (08.05.03), Full text; particularly, page 1, 58 to 60<br>& EP 144072 A2 & US 2005049263 A1<br>& JP 2005-511565 A | 1-21 |
| A | JP 10-152477 A (Pfizer Products Inc.), 09 June, 1998 (09.06.98), Full text<br>& EP 837063 A1 & CA 2218945 A<br>& BR 9705088 A & MX 212865 B<br>& US 6225318 B1 & JP 345764 B2 | 1-21 |
| A | JP 2004-501139 A (Pfizer Products Inc.), 15 January, 2004 (15.01.04), Full text<br>& WO 01/098277 A2 & AU 200164159 A<br>& NO 200206166 A & EP 1292591 B1<br>& BR 200111548 A & KR 2003016303 A<br>& HU 200301120 A2 & CN 1437594 A<br>& ZA 200210231 A & SK 200201710 A3<br>& CZ 200203951 A3 & NZ 522568 A<br>& US 6890924 B2 | 1-21 |
| A | JP 2003-509499 A (Astra Zeneca AB.), 11 March, 2003 (11.03.03), Full text<br>& WO 01/021596 A1 & AU 200073010 A<br>& NO 200201399 A & EP 1218354 A1<br>& BR 20014116 A & KR 2002032612 A<br>& CZ 200201009 A3 & HU 200300059 A2<br>& CN 1391562 A & ZA 200202234 A<br>& SK 200200382 A3 | 1-21 |

Form PCT/ISA/210 (continuation of second sheet) (January 2004)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0266445 A
- WO 9633980 A
- US 6083903 A
- JP 2004254716 A


### Non-patent literature cited in the description


- *Cancer Res.,* 2001, vol. 61, 1799-804
- *Int. J. Cancer,* 1992, vol. 51, 613-9
- *Nature,* 2003, vol. 425, 407-10
- *J. Natl. Cancer Inst.,* 1993, vol. 85, 570-4
- *Clin. Cancer Res.,* 1999, vol. 5, 1650-7
- *Pathol. Oncol. Res.,* 1998, vol. 4, 316-21
- *Cancer Cell,* 2003, vol. 3, 213-7
- **KUREBAYASHI, J. et al.** *British J Cancer,* 1999, vol. 79, 707-717
- *Cancer Res.,* 2002, vol. 62, 2474-2477